# EUROPÄISCHE PATENTSCHRIFT

(11) **EP 2 174 645 B1**
(45) Veröffentlichungstag und Bekanntmachung des Hinweises auf die Patenterteilung: **29.07.2015**
(21) Anmeldenummer: 09173453.3
(22) Anmeldetag: 19.10.2009
(51) Int. Cl.: A61K 8/37, A61Q 13/00

(54) **Riechstoffhaltige Zusammensetzungen umfassend Cetylnonanoat und/oder Stearylnonanoat**
Materials containing aromas incorporating cetyl nonanoate and/or stearyl nonanoate
Compositions à teneur en parfum comprenant du cétylnonanoate et/ou du stearylnonanoate

(43) Veröffentlichungstag der Anmeldung: 14.04.2010
(73) Patentinhaber: Symrise AG, 37603 Holzminden (DE)
(72) Erfinder: Wöhrle, Ingo, 28203, Bremen (DE); Kuhn, Walter, 37603, Holzminden (DE); Meier, Manfred, 37699, Fürstenberg (DE); Schmaus, Gerhard, 37671, Höxter-Bosseborn (DE)
(74) Vertreter: Eisenführ Speiser

(56) Entgegenhaltungen:
- WO-A2-2008/027434
- Boevé J-L, Heilporn S, Dettner K, Francke W: "The secretion of the ventral glands in Cladius, Priophorus and Trichiocampus sawfly larvae" Biochemical Systematics and Ecology Bd. 28, Nr. 9, 2000, Seiten 857-864, XP002574290 Gefunden im Internet: URL:http://www.sciencedirect.com/science?_ ob=ArticleListURL&_method=list&_ArticleLis tID=1231051450&_sort=d&_st=0&_acct=C000049 880&_version=1&_urlVersion=0&_userid=98776 6&md5=7495a7f643803c667f27f9bab8e345e1>

## Beschreibung

Die Erfindung betrifft die Verwendung von n-Hexadecyl n-nonanoat (CAS Nummer 72934-15-7, im Folgenden Cetylnonanoat) und n-Octadecyl n-nonanoat (CAS Nummer 107647-13-2, im Folgenden Stearylnonanoat) sowie deren Mischungen (CAS Nummer 878027-13-5) als Fixateure für Riechstoffe. Ferner betrifft die Erfindung bestimmte Zusammensetzungen enthaltend (A) Cetylnonanoat und/oder Stearylnonanoat sowie (B) ein oder mehrere Riechstoffe. Die Erfindung betrifft ferner ein Verfahren zur Herstellung solcher Zusammensetzungen sowie (kosmetische) Produkte enthaltend eine erfindungsgemäße Zusammensetzung. Ferner betrifft die Erfindung ein Verfahren zur Vermittlung, Verstärkung oder Modifizierung eines Geruchs auf der (menschlichen) Haut. Ein besonderer Aspekt der vorliegenden Erfindung betrifft die Verbesserung der Haftfestigkeit einer erfindungsgemäßen Zusammensetzung auf (menschlicher) Haut und/oder (menschlichem) Haar.

Im Bereich der Riechstoffe und der Parfümerie ist es allgemein bekannt, dass es bei Anwendung von Riechstoffzusammensetzungen auf der Haut durch die Verdampfung von Verdünnungs- oder Lösemitteln zu einer Abnahme von Riechstoffen, insbesondere der leichtflüchtigen Kopfnote (Topnote), kommt, während die schwerer flüchtigen Noten eine hervorragende Haftung auf der Haut aufweisen und über einen langen Zeitraum von der Haut abgegeben und dementsprechend geruchlich länger wahrgenommen werden. Durch die schnellere Verdampfung der Kopfnote kommt es folglich im zeitlichen Verlauf zu einer deutlichen Veränderung des Geruchsprofils von auf Haut aufgetragenen Riechstoffzusammensetzungen. Ein ähnlicher Effekt tritt bei der Anwendung Riechstoffzusammensetzungen auf (menschlichem) Haar auf. Hierbei ist zu berücksichtigen, dass der beschriebene nachteilige Effekt insbesondere dann als problematisch empfunden wird, wenn eine Riechstoffzusammensetzung (Riechstoffmischung) als leave-on-Produkt eingesetzt wird, das heißt auf der Haut und/oder dem Haar verbleiben soll, um von dort aus über einen längeren Zeitraum abgegeben zu werden.

Unter Haftung, Haftfestigkeit, Haftbarkeit und Substantivität wird im Rahmen des vorliegenden Textes die Haftung eines Riechstoffes auf Haut und Haar, insbesondere die Haftung auf menschlicher Haut (ausschließlich der (Mund)Schleimhaut) verstanden. Wenngleich also die vorliegende Erfindung hinsichtlich der Applikation von Riechstoffzusammensetzungen auch die (Mund)Schleimhaut betrifft, ist doch festzustellen, dass die Bedeutung der vorliegenden Erfindung hinsichtlich der Aspekte Haut (ausschließlich (Mund)Schleimhaut) und/oder Haar (insbesondere menschliche Haut) von noch weit höherer Relevanz ist, denn bei Applikation einer Riechzusammensetzung auf die (Mund)Schleimhaut wird regelmäßig der negative Verdampfungseffekt überlagert und dominiert von Abwaschungseffekten (im Bereich der Mundschleimhaut beispielsweise unterstützt durch den Einfluss von Speichel).

In der parfümistischen Praxis wurden bereits zahlreiche Versuche unternommen, um die Haftbarkeit bzw. die Wahrnehmbarkeit von Riechstoffzusammensetzungen, insbesondere auf (menschlicher) Haut, zu verlängern und somit eine gewisse geruchliche zeitliche "Profilstabilität" zu erreichen.

Ein Fixateur erhöht die Haftfestigkeit von Riechstoffen, z.B. durch deren Dampfdruckerniedrigung. Unter Fixateuren versteht man hierbei solche Stoffe, die eine zeitlich verzögerte Freisetzung der Parfümölbestandteile, beispielsweise auf Haut und/oder Haar, ermöglichen und somit einen länger anhaltenden Dufteindruck gewährleisten.

Besonders geeignet sind hierbei solche Fixateure, die geruchlos sind oder über einen sehr geringen Eigengeruch verfügen, so dass sie den Geruchseindruck von Riechstoffen, Riechstoffmischungen und Parfümölen nicht verändern.

Die Verwendung von Fixateuren ist vielfach beschrieben, d.h. die Verwendung von Einzelstoffen oder Kombinationen von (Riech)Stoffen, die leichter flüchtigen Riechstoffen oder Riechstoffmischungen zugesetzt werden, um deren Verdampfungsgeschwindigkeit zu reduzieren (vgl. US 6,737,396).

In IP.COM #000033581D (veröffentlicht Januar 2005) wird die Verwendung von Hydroxyalkylharnstoff-Derivaten, insbesondere Hydroxyethylharnstoff, beschrieben, wodurch die Haftungseigenschaften von Parfüms in kosmetischen Anwendungen, insbesondere wässrig-ethanolischen Formulierungen, auf Haut und Haar verlängert wird.

In US 3,939,099 wird die Verwendung von Filmbildnern beschrieben, die sich in einem Wasser/Ethanolgemisch lösen und mit Riechstoff(mischungen) mischbar sind. Als Beispiele werden ionische und nichtionische Derivate von wasserlöslichen Polymeren genannt wie z.B. Polyvinylpyrrolidonderivate, quarternäre Polyvinylpyrrolidone mit Molgewichten im Bereich 50000 - 1000000, kationische Cellulosederivate und ähnliche. Während des Verdampfens von Ethanol kommt es zur Bildung eines Films auf der Haut, in den die Riechstoffe eingelagert sind.

US 6,210,688 beschreibt die Bildung und Verwendung eines geruchlosen Polymerfilms auf der Haut (basierend auf Vinylether-Copolymeren, Polyacrylaten, Methacrylaten, Polyestern, Polyfluorkohlenwasserstoffen, Polysaccariden), auf den anschließend ein Parfüm appliziert wird. Dadurch sollen "Reaktionen" mit der Haut unterbunden werden.

FR 2 747 306 beschreibt die Verwendung polymerer Kohlenwasserstoffe (Polyethylene mit Molekulargewichten zwischen 3000 und 30000). Da diese Polymere in Ethanol/Wasser unlöslich sind, ist ein entsprechendes Produkt wie z.B. Eau de Cologne, EdT oder ein Eau de Parfum trübe.

WO 2004/098556 beschreibt eine neuartige sprühbare und klare Parfümformulierung, die sich durch eine erhöhte Oberflächenspannung bzw. verringerte Berührungsfläche nach Applikation auszeichnet, die durch die Anwendung einer wirksamen Menge eines Polymers erreicht wird. Durch die kleinere Berührungsfläche wird das verbleibende Parfümöl nach dem Verdampfen des Ethanols auf einer kleineren Fläche konzentriert, von der die Riechstoffe langsamer abdampfen.

Filmbildner und/oder Polymere haben den Nachteil, dass sie nicht nur die Verdampfungsgeschwindigkeit der Kopfnote, sondern auch die aller anderen, schwerer flüchtigen Riechstoffe reduzieren, wodurch die Gesamtintensität merklich reduziert wird. Weiterhin können Filmbildner ein unangenehmes klebriges bzw. spannendes Hautgefühl hervorrufen.

EP 0 181 401 und EP 0 857 481 beschreiben gelartige Parfümzubereitungen, in denen die Diffusion und damit auch die Verdampfung von Riechstoffen reduziert wird. Die vorgeschlagene Gelbildung ist allerdings insbesondere für manche Anwendungsbereiche, wie beispielsweise der Feinparfümerie oder Produkten in Form von Lotionen oder Sprays nicht geeignet, da die dort beschriebenen gelartigen Zubereitungen zu viskos sind.

EP 1 872 831 beschreibt bestimmte ethanolische Riechstoffzusammensetzungen enthaltend deliqueszente Stoffe zur Vermittlung, Verstärkung oder Modifizierung eines Geruchs, dabei insbesondere die Verbesserung der Haftfestigkeit von ethanolischen Riechstoffzusammensetzungen auf (menschlicher) Haut und/oder (menschlichem) Haar.

Die gesuchten Fixateure sollen den obigen Anforderungen gerecht werden und mit möglichst vielen gängigen (Parfümerie-)Riechstoffen und Parfümölen gut mischbar und darüber hinaus in eine Vielzahl kosmetischer Formulierungen eingearbeitet werden können.

Es war daher die primäre Aufgabe der vorliegenden Erfindung, eine (alternative) Riechstoffzusammensetzung anzugeben, in der die eingesetzten Riechstoffe, vorzugsweise Riechstoffe, welche die Kopfnote und/oder die Fondnote eines Duftes bilden, eine lange (gegenüber konventionellen Riechstoffzusammensetzungen verlängerte) Haftung (Haftfestigkeit) insbesondere auf (menschlicher) Haut (damit ist Haut exklusive Schleimhaut gemeint) und/oder (menschlichem) Haar besitzen. Dabei sollten die vorstehend geschilderten Nachteile bisheriger Riechstoffzusammensetzungen möglichst vermieden werden.

Die primäre gestellte Aufgabe wird erfindungsgemäß gelöst durch eine Zusammensetzung umfassend (oder bestehend aus):
(A) Cetylnonanoat und/oder Stearylnonanoat,
   und
(B) einem oder mehreren Riechstoffen, vorzugsweise einer Riechstoffmischung umfassend 2, 3, 4, 5, 6, 7, 8, 9, 10 oder mehr Riechstoffe,
wobei Bestandteil (A) in einer für den, mehrere oder sämtliche Riechstoffe des Bestandteils (B) fixierenden Menge enthalten ist, wobei vorzugsweise das Massenverhältnis des Bestandteils (A) zur Gesamtmasse des Bestandteils (B) im Bereich von 1 : 20 bis 200 : 1 liegt, bevorzugt im Bereich von 1 : 12 bis 100 : 1, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1.

Unter einer fixierenden Menge des Bestandteils (A) ist eine Menge an Bestandteil (A) zu verstehen, die ausreicht, eine Fixierung eines, mehrerer oder sämtlicher Riechstoffe des Bestandteils (B) zu bewirken.

Die erfindungsgemäßen Zusammensetzungen zeigen überraschenderweise eine Fixierung des, der oder sämtlicher Riechstoffe des Bestandteils (B) durch den Bestandteil (A), insbesondere bei Einstellung der als bevorzugt bezeichneten Massenverhältnissen. Durch eine fixierend wirksame Menge des Bestandteils (A), der als Fixateur für einen, mehrere oder sämtliche Riechstoffe des Bestandteils (B) wirkt, wird eine verbesserte und verlängerte Haftung (Haftfestigkeit) des Bestandteils (B) erreicht, insbesondere auf (menschlicher) Haut und/oder (menschlichem) Haar. Dies gilt ebenfalls für erfindungsgemäße kosmetische Produkte, die vorzugsweise eine sensorisch (geruchlich) wirksame Menge einer erfindungsgemäßen Zusammensetzung enthalten.

In der Veröffentlichung zum 7. Int. Congr. Essent. Oils, Kyoto 1977, October 7-11, Volume 7, 461-466 (1979) wurde das Concrete von Bulgarischer Rose (*Rosa damascena* Mill.) analytisch untersucht. Neben zahlreichen Riechstoffen wurden zahlreiche Esterkomponenten identifiziert, darunter als nicht quantifizierte Spurenkomponente auch Cetylnonanoat. Stearylnonanoat wurde dabei nicht gefunden. In einer Ausführungsform ist eine erfindungsgemäße Zusammensetzung kein Concrete von Bulgarischer Rose, insbesondere nicht um ein Concrete von Bulgarischer Rose wie in diesem Dokument beschrieben. In einer weiteren bevorzugten Ausführungsform enthält eine erfindungsgemäße Zusammensetzung bzw. ein erfindungsgemäßes kosmetisches Produkt kein Concrete von Bulgarischer Rose. In einer weiteren bevorzugten Ausführungsform enthält eine erfindungsgemäße Zusammensetzung bzw. ein erfindungsgemäßes kosmetisches Produkt kein Concrète von *Rosa damascena.*

Gemäß Biochemical Systematics and Ecology 2000, 28(9), 857-864 wurde Cetylnonanoat in einem Anteil von 1 GC-% im Drüsenextrakt der Wespenart *Trichiocampus grandis* gefunden, der unter anderem Spuren des Riechstoffs (Z)-3-Octenal enthält. Ein solcher Extrakt ist nicht Gegenstand der vorliegenden Erfindung. In einer bevorzugten Ausführungsform handelt es sich bei einer erfindungsgemäßen Zusammensetzung nicht um einen Drüsenextrakt von *Trichiocampus grandis*, insbesondere nicht um einen Drüsenextrakt wie in obigem Dokument beschrieben. In bevorzugten Ausführungsformen ist eine erfindungsgemäße Zusammensetzung entweder frei von (Z)-3-Octenal (welches kein Parfümerie-Riechstoff gemäß untenstehender Definition ist) oder sie enthält neben (Z)-3-Octenal zumindest einen weiteren Riechstoff, bevorzugt zwei, drei, vier, fünf oder mehr weitere Riechstoffe, vorzugsweise Parfümerie-Riechstoffe (wie unten definiert).

In Revue Française des Corps Gras 1986, 33(11), 423-430 wird das Mycel (mycelium) des (pathogenen) Pilzes *Rhizopus arrhizus* auf seine Lipase-Aktivität hin untersucht. Dort wird unter anderem die Veresterung eines C18-Alkohols (möglicherweise Stearylalkohol) mit einer C9-Carbonsäure (möglicherweise n-Nonansäure) in Methyl-tert.-butylether (MTBE) in Gegenwart der Mycelien von *Rhizopus arrhizus* und Molekularsieb beschrieben. Unbekannt ist, ob *Rhizopus arrhizus* Riechstoffe enthält. Eine Mischung wie in Revue Française des Corps Gras 1986, 33(11), 423-430 beschrieben ist nicht Gegenstand der vorliegenden Erfindung. Vorsorglich ist in bevorzugten Ausführungsformen eine erfindungsgemäße Zusammensetzung frei von MTBE, Molsieb und/oder Mycelien von *Rhizopus arrhizus.*

In der Veröffentlichung zum 8. International Symposium on Chemical Signals in Vertebrates, Ithaca, NY, Juli 20-25, 1997, veröffentlicht 1999 in Advances in Chemical Signals in Vertebrates, 1999, 163-171, Kluwer Academic/Plenum Publishers, New York, N. Y. wird beschrieben, dass Cetylnonanoat und Stearylnonanoat in einem Drüsensekret der rotbraunen Elefantenspitzmaus *Elephantus rufescens* (engl.: rufous elephant-shrew), das unter anderem auch riechende Substanzen wie Nonanal enthält. Es wurden dort keine Angaben zu Mengen oder Mengenverhältnissen der Drüsensekretsbestandteile gemacht. Ein solches Sekret ist nicht Gegenstand der vorliegenden Erfindung. Dementsprechend handelt es sich bei einer erfindungsgemäßen Zusammensetzung nicht um ein Drüsensekret von *Elephantus rufescens,* insbesondere nicht um ein Drüsensekret wie in obigem Dokument beschrieben.

### Bestandteil (A)

Cetylnonanoat (Hexadecyl nonanoat; CAS Nummer 72934-15-7; nachfolgende Formel I) und Stearylnonanoat (Octadecyl nonanoat; CAS Nummer 107647-13-2, nachfolgende Formel II) entsprechen folgenden Strukturformeln:

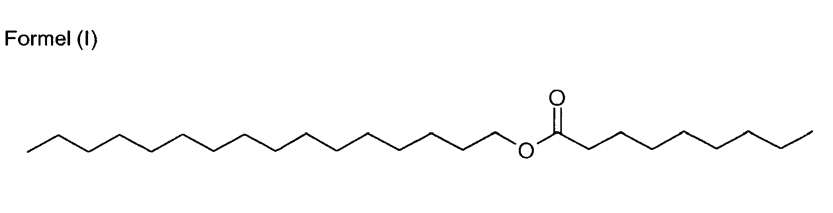

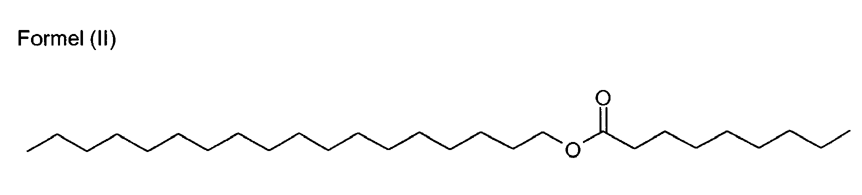

Cetylnonanoat und Stearylnonanoat sowie deren Mischungen (CAS Nummer 878027-13-5) können beispielsweise mit klassischen chemischen oder biotechnologischen Veresterungsverfahren durch Umsetzung von Cetlyalkohol und/oder Stearylalkohol hergestelt werden. Ferner sind sie kommerziell erhältlich, ebenso wie deren Mischungen.

Vorzugsweise enthalten erfindungsgemäße Zusammensetzungen Cetylnonanoat und Stearylnonanoat. Vorzugsweise liegt dabei das Massenverhältnis von Cetylnonanoat zu Stearylnonanoat im Bereich von 1 : 9 bis 9 : 1, bevorzugt im Bereich von 2 : 8 bis 8 : 2, weiter bevorzugt im Bereich von 3 : 7 bis 7 : 3, da solche Zusammensetzung verbesserte geruchliche Effekte im oben beschriebenen Sinne zeigen.

Bestandteil (A) einer erfindungsgemäßen Zusammensetzung eignet sich hervorragend für die gestellte Aufgabe, da Cetylnonanoat und Stearylnonanoat sowie deren Mischungen keinen oder allenfalls sehr geringen Eigengeruch aufweisen. Bestandteil (A) ist selbstverständlich nicht Teil des Bestandteils (B).

Ferner weisen Cetylnonanoat und Stearylnonanoat sowie insbesondere deren Mischungen, vorzugsweise in den oben genannten Gewichtsverhältnissen, einen Schmelzbereich auf, der der menschlichen Hauttemperatur nahe ist, so dass solche Mischungen ein angenehmes Hautgefühl vermitteln, wodurch erfindungsgemäße Zusammensetzung hervorragend für die Applikation auf (menschliche) Haut eignen sowie für die Einarbeitung und als Teil von kosmetischen (topischen) Produkten.

### Bestandteil (B)

Beispiele für Riechstoffe des Bestandteils (B), ebenso der (bevorzugten) Bestandteile (B*), (B) (i) und (B) (ii), sind dem Fachmann bekannt und beispielsweise zu finden in S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Vorzugsweise handelt es sich bei Bestandteil (B) um eine Riechstoffmischung, bevorzugt um eine Riechstoffmischung umfassend 3, 4, 5, 6, 7, 8, 9, 10 oder mehr Riechstoffe, weiter bevorzugt um Parfümerie-Riechstoffe, insbesondere um ein Parfümöl.

Bevorzugte Riechstoffe des Bestandteils (B) sind Parfümerie-Riechstoffe, welche wiederum bevorzugt gewählt sind aus der Gruppe (B*) bestehend aus der Gruppe der als Riechstoffe offenbarten Einzelverbindungen gemäß
- S. Arctander, Perfume and Flavor Materials, Vol. I und II, Montclair, N. J., 1969, Selbstverlag ("Arctander"),
- H. Surburg, J. Panten, "Common Fragrance and Flavor Materials", 5th. Ed., Wiley-VCH, Weinheim 2006 ("Surburg"),
   und
- den im Rahmen des vorliegenden Textes als Riechstoffe bezeichneten Einzelverbindungen.

Sollte im Einzelfall eine mangelnde Übereinstimmung oder ein Widerspruch zwischen diesen drei Stellen bestehen, hat der vorliegende Text Vorrang vor "Arctander" und "Surburg". Sollte im Einzelfall eine mangelnde Übereinstimmung oder ein Widerspruch zwischen "Surburg" und "Arctander" bestehen, hat "Surburg" Vorrang vor "Arctander".

In einer bevorzugten Ausgestaltung betrifft die vorliegende Erfindung eine Zusammensetzung umfassend (oder bestehend aus):
(A) Cetylnonanoat und/oder Stearylnonanoat,
   und
(B) einem oder mehreren Riechstoffen der Gruppe (B*) wie oben definiert, vorzugsweise einer Riechstoffmischung umfassend 2, 3, 4, 5, 6, 7, 8, 9, 10 oder mehr Riechstoffe der Gruppe (B*),
   wobei das Massenverhältnis des Bestandteils (A) zur Gesamtmasse des Bestandteils (B*) im Bereich von 1 : 20 bis 200 : 1, bevorzugt im Bereich von 1 : 12 bis 100 : 1, liegt, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1, liegt.

Weiter bevorzugte erfindungsgemäße Zusammensetzungen sind solche, bei denen Bestandteil (B), vorzugsweise Bestandteil (B*),
(B) (i) einen oder mehrere Riechstoffe mit einem Molgewicht im Bereich von 100 g/mol bis 175 g/mol (Topnote) enthält, vorzugsweise mit einem Molgewicht im Bereich von 110 g/mol bis 160 g/mol, bevorzugt im Bereich von 115 g/mol bis 160 g/mol, besonders bevorzugt im Bereich von 120 g/mol bis 155 g/mol,
   und/oder
(B) (ii) einen, 2, 3, 4, 5 oder mehr Riechstoffe mit einem Molgewicht von größer oder gleich 190 g/mol (Fondnote) enthält, vorzugsweise mit einem Molgewicht im Bereich von 190 g/mol bis 300 g/mol, bevorzugt mit einem Molgewicht im Bereich von 195 g/mol bis 290 g/mol, und besonders bevorzugt im Bereich 200 bis 275 g/mol.

In einer bevorzugten Ausführungsform enthält eine erfindungsgemäße Zusammensetzung zumindest 2, 3, 4, 5 oder mehr Riechstoffe des Bestandteils (B) (i), und/oder zumindest 2, 3, 4, 5 oder mehr Riechstoffe des Bestandteils (B) (ii).

Erfindungsgemäße Zusammensetzungen, die einen oder mehrere (als bevorzugt bezeichnete) Riechstoffe des Bestandteils (B) (i), insbesondere in den nachfolgend angegebenen Mengenanteilen, und einen oder mehrere Riechstoffe des Bestandteils (B) (ii) enthalten, zeigen darüberhinaus eine geruchliche Harmonisierung und Abrundung, wodurch ein eleganterer und somit geruchlich wertvollerer Geruchseindruck entsteht. Dies gilt insbesondere für die Moschusriechstoffe und weiteren nachfolgend als bevorzugt bezeichneten Fondnoten des Bestandteils (B) (ii), insbesondere in den nachfolgend angegebenen Mengenanteilen.

Die (bevorzugten) Riechstoffe der Bestandteile (B), (B*), (B) (i) sowie (B) (ii) können dabei, sofern zutreffend, in Form ihrer jeweiligen Diastereomere, Enantiomere und/oder Doppelbindungsisomere vorliegen. So können diese als (E)/(Z)-Isomere, als beliebiges Gemisch der Enantiomeren, insbesondere als Racemat, oder auch als beliebiges Gemisch der entsprechenden Diastereomeren, vorliegen.

### Bestandteil (B) (i)

Die Riechstoffe des Bestandteils (B) (i) sind als Kopfnoten (Topnoten) einer erfindungsgemäßen Zusammensetzung bzw. eines erfindungsgemäßen kosmetischen Produktes anzusehen. Die Kopfnote bestimmt den Anfangsgeruch (Angeruch) einer Riechstoffmischung bzw. eines Parfümöls.

Das Molgewicht der Riechstoffe des Bestandteils (B) (i) liegt im Bereich von 100 g/mol bis 175 g/mol (Kopfnote), vorzugsweise im Bereich von 110 g/mol bis 160 g/mol, bevorzugt im Bereich von 115 g/mol bis 160 g/mol, und besonders bevorzugt im Bereich von 120 g/mol bis 155 g/mol.

Vorzugsweise umfasst Bestandteil (B) (i) einer erfindungsgemäßen Zusammensetzung (insbesondere in einer der als bevorzugt gekennzeichneten Ausgestaltungen) einen oder mehrere Riechstoffe aus der Gruppe bestehend aus:
n-Heptanol, Campher, alpha-Pinen, beta-Pinen, Limonen, 6-Methyl-5-hepten-2-on, Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Citral, Neral, Geranial, Benzylalkohol, p-Anisaldehyd, Menthol, Isoamylacetat, Isoamylbutyrat, cis-3-Hexenylacetat, Hexylacetat, Butylbutyrat, Citronellol, Nerol, Geraniol, 2-Phenylethylalkohol, Methylbenzoat, Agrunitril (3,7-Dimethyl-6-octen-1-nitril) und Vanillin.

Besonders bevorzugt sind folgende Riechstoffe des Bestandteils (B) (i), da bei diesen eine höhere Retention erzielt wurde: n-Heptanol, Limonen, 6-Methyl-5-hepten-2-on, Octanal, Rosenoxid, Dihydromyrcenol (2,6-Dimethyl-7-octen-2-ol), 2,6-Dimethyl-5-hepten-1-al (Melonal), 3-Hexenylmethylcarbonat, Linalool, Citral, Neral, Geranial, p-Anisaldehyd, Hexylacetat, Citronellol, Nerol, Geraniol, 2-Phenylethylalkohol, Methylbenzoat, Agrunitril (3,7-Dimethyl-6-octen-1-nitril) und Vanillin. Bei Einsatz der genannten Riechstoffe der Gruppe (B) (i) sind die genannten Effekte besonders ausgeprägt.

Die Gesamtmenge des oder der (als bevorzugt bezeichneten) Riechstoffe des Bestandteils (B) (i) (Kopfnote) einer erfindungsgemäß bevorzugten Zusammensetzung bzw. eines erfindungsgemäß bevorzugten kosmetischen Produktes beträgt 5 bis 80 Gew.-%, besonders bevorzugt 10 bis 70 Gew.-%, jeweils bezogen auf das Gesamtgewicht des Bestandteils (B), vorzugsweise jeweils bezogen auf das Gesamtgewicht des Bestandteils (B*).

### Bestandteil (B) (ii)

Die Riechstoffe des Bestandteils (B) (ii) sind als Fondnoten einer erfindungsgemäßen Zusammensetzung bzw. eines erfindungsgemäßen kosmetischen Produktes anzusehen. Die Fondnote bestimmt den Nachgeruch einer Riechstoffmischung bzw. eines Parfümöls.

Das Molgewicht der Riechstoffe des Bestandteils (B) (ii) ist größer oder gleich 190 g/mol und liegt bevorzugt im Bereich von 190 g/mol bis 300 g/mol, weiter bevorzugt im Bereich von 195 g/mol bis 290 g/mol, und am meisten bevorzugt im Bereich von 200 bis 275 g/mol.

Mit solchen Riechstoffen des Bestandteils (B) (ii) können die beschriebenen Effekte der vorliegenden Erfindung am ausgeprägtesten und deutlichsten erreicht werden Bevorzugte erfindungsgemäße Zusammensetzungen sind solche, in denen Bestandteil (B) (ii) einen, zwei, drei oder mehr Riechstoffe enthält gewählt aus der Gruppe bestehend aus:

### - Moschusriechstoffen,

alpha-n-Amylzimtaldehyd (MW = 202,30), alpha-iso-Amylzimtaldehyd (MW = 202,30), alpha-n-Hexylzimtaldehyd (MW = 216,32), alpha-iso-Hexylzimtaldehyd (MW = 216,32), Benzylsalicylat (MW = 228,25), cis-3-Hexenylsalicylat (MW = 220,27), Isoamylsalicylat (MW = 208,26), Hexylsalicylat (MW = 222,28), 2-Methyl-3-(4-tert-butylphenyl)propanal (MW = 204,31; Lilial®), 2-Methyl-3-(4-isopropylphenyl)propanal (MW = 190,28, Cyclamenaldehyd), 2,3,8,8-Tetramethyl-1,2,3,4,5,6,7,8-octahydro-2-naphthalenylmethylketon (MW = 234,38, Iso E Super®), 1-[1,2,3,4,6,7,8,8a-octahydro-1,2,8,8-tetramethylnaphtalen-2-yl]ethanon (MW = 234,38, Iso E Super®), Methyldihydrojasmonat (MW = 226,32, Hedione®), Linalylacetat (MW = 196.29), Ethyllinalylacetat (MW = 210,31), Nerolidol (MW = 222,37), Farnesol (MW = 222,37), Cedrylmethylether (MW = 236,40, Cedramber), Cedrylmethylketon (MW = 246,39), Cedrylacetat (MW = 264,41), (4aR,5R,7aS,9R)-Octahydro-2,2,5,8,8,9a-hexamethyl-4H-4a,9-Methanoazuleno(5,6-d) 1,3-dioxol) (MW = 278,44, Ambrocenide®), Hexahydro-1',1',5',5'-tetramethyl-spiro[1,3-dioxolan,2,8' (5'H)-[2H-2,4a]-methanonaphthalin (MW = 264,41 Ethylendioxi-3H-isolongifolan, Ysamber® K), 2-Methyl-4-(2,2,3-trimethyl-3-cyclopenten-1-yl)butanol (MW = 196,34, Brahmanol), 5-(2,2,3-Trimethyl-3-cyclopenten-1-yl)-3-methylpentan-2-ol (MW = 210,36, Sandalore®), 2-Ethyl-4-(2,2,3-trimethyl-3-cyclopenten-1-yl)-2-buten-1-ol (MW = 208,35, Sandranol®), 3-Methyl-5-(2,2,3-trimethyl-3-cyclopenten-1-yl)-4-penten-2-ol (MW = 208,35, Ebanol®), 3,3-Dimethyl-5-(2,2,3-trimethyl-3-cyclopenten-1-yl)-4-penten-2-ol (MW = 222,37, Polysantol®), 3-Isocamphylcyclohexanol (MW = 236,40, Sandel 80®),1-(2,2,6-Trimethylcyclohexyl)hexan-3-ol (MW = 226,41, Timberol®), Cyclododecylmethylether (MW = 198,35, Palisandin), (Ethoxymethoxy)cyclododecan (MW = 242,41, Boisambrene forte®), 1-Methyl-4-(4-methyl-3-penten-1-yl)-3-cyclohexencarboxaldehyd (MW = 206,33, Precyclemone B®), 4-(4-Hydroxy-4-methylpentyl)-3-cyclohexencarboxaldehyd (MW = 210,32, Lyral®), 2-Methyl-4-(2,2,6-trimethyl-1-cyclohexen-1-yl)-2-butenal (MW = 206,33, Boronal), Decahydro-beta-naphthylacetat (MW = 196,29), Allyl-3-cyclohexylpropionat (MW = 196,29), Allylcyclohexyloxyacetat (MW = 198,26, Isoananat®), Citraldiethylacetal (MW = 226,36), Benzylbenzoat (MW = 212,25), Benzylcinnamat (MW = 238,29), 3a,6,6,9a-Tetramethyldodecahydronaphtho[2,1-*b*]furan (MW = 236,40, Ambroxid®), alpha-Iron (MW = 206,33), beta-Iron (MW = 206,33), alpha-n-Methylionon (MW = 206,33), beta-n-Methylionon (MW = 206,33), alpha-Isomethylionon (MW = 206,33), beta-Isomethylionon (MW = 206,33) und Allylionon (MW 232,35). 2,3,8,8-Tetramethyl-1,2,3,4,5,6,7,8-octahydro-2-naphthalenylmethylketon (MW = 234,38), 1-[1,2,3,4,6,7,8,8a-octahydro-1,2,8,8-tetramethylnaphtalen-2-yl]ethanon (MW = 234,38), Isobornylacetat (MW = 196,29), alpha-Ionon (MW = 192,30), beta-Ionon (MW = 192,30), gamma-Ionon (MW = 192,30), alpha-Damascon (MW = 192,30), beta-Damascon (MW = 192,30), delta-Damascon (MW = 192,30), 1-(2,4,4-Trimethyl-2-cyclohexen-1-yl)-2-buten-1-on (MW = 192,30, Isodamascon), Cedrol (MW = 222,40), gamma-Dodecalacton (MW = 198,30), 2-Methyl-3-(3,4-methylendioxyphenyl)propanal (MW = 192,22, Helional) und Methyldihydrojasmonat (MW = 226,31).

In Klammern sind dabei die Molgewichte (MW) und ggf. übliche Marken- bzw. Produktnamen angegeben.

Der oder die Moschusriechstoffe des Bestandteils (B) (ii) sind Riechstoffe, die einen Moschusgeruch aufweisen. Solche Riechstoffe sind dem Fachmann bekannt, da "Moschus" (musk) eine sehr wichtige Geruchsrichtung in der Parfümerie darstellt. Ferner sind Moschusriechstoffe, unter anderem die unten bevorzugt genannten, beschrieben in H. Surburg, J. Panten, "Common Fragrance and Flavor Materials", 5th. Edition, Wiley-VCH, Weinheim 2006.

Der oder die Moschusriechstoffe, die Teil des Bestandteils (B) (ii) sind, sind dabei vorzugsweise gewählt aus der Gruppe der macrocyclischen Moschusriechstoffe, der polycyclischen Moschusriechstoffe und/oder der alicyclischen Moschusriechstoffe.

Erfindungsgemäß bevorzugt sind Bestandteile (B), insbesondere Parfümerie-Riechstoffmischungen, enthalten zwei, drei oder mehrere verschiedene Moschusriechstoffe als Bestandteil (B) (ii).

Bevorzugt werden der oder die Moschusriechstoffe des Bestandteils (B) (ii) im Rahmen der vorliegenden Erfindung gewählt aus nachfolgender Tabelle 1:

**Tabelle 1:**

| TYP | Produkt / Markenname | Name / CAS-Name |
|---|---|---|
| MACRO | EXALTENON | 4-Cyclopentadecen-1-one (4Z)- ; 4-Cyclopentadecen-1-on |
| MACRO | CIVETON | 9-Cycloheptadecen-1-on, (9Z)- |
| MACRO | CYCLOHEXADECANOLID, DIHYDROAMBRETTOLID | Oxacycloheptadecan-2-on, ω-Hexadecanolid |
| MACRO | ETHYLENDODECANDIOAT | 1,4-Dioxacyclohexadecane-5,16-dion |
| MACRO | GLOBALIDE® | Oxacyclohexadecen-2-on; 15-Pentadec-(11/12)-enolid |
| MACRO | ETHYLENBRASSYLAT | 1,4-Dioxacycloheptadecane-5,17-dion |
| MACRO | MUSCON | 3-Methy-cyclopentadecanon |
| MACRO | AMBRETTOLID | Oxacycloheptadec-10-en-2-on |
| MACRO | MUSCENON | 3-Methyl-cyclopentadecenon |
| MACRO | VELVIONE®, AMBRETONE | 5-Cyclohexadecen-1-on |
| MACRO | AURELIONE® | 7/8-Cyclohexadecen-1-on |
| MACRO | GLOBANONE® | 8-Cyclohexadecen-1-on |
| MACRO | ISOMUSCONE® | Cyclohexadecanon |
| MACRO | EXALTOLID, MACROLIDE® | Oxacyclohexadecan-2-on |
| MACRO | COSMONE® | 3-Methyl-(5E/Z)-cyclotetradecen-1-on |
| POLY | TRASEOLIDE® | 1-[2,3-Dihydro-1,1,2,6-tetramethyl-3-(1-methylethyl)-1H-inden-5-yl]-ethanon |
| POLY | PHANTOLIDE® | 1-(2,3-Dihydro-1,1,2,3,3,6-hexamethyl-1H-inden-5-yl)-ethanon |
| POLY | TONALIDE® | 1-(5,6,7,8-Tetrahydro-3,5,5,6,8,8-hexamethyl-2-naphthalenyl)-ethanon |
| POLY | CRYSOLIDE | 1-[6-(1,1-Dimethylethyl)-2,3-dihydro-1,1-dimethyl-1H-inden-4-yl]-ethanon |
| POLY | CHROMANOLIDE® | Tetradecansäure, 1-methylethyl ester; Cyclopenta[g]-2-benzopyran, 1,3,4,6,7,8-hexahydro-4,6,6,7,8,8-hexamethyl- |
| POLY | GALAXOLIDE® | Cyclopenta[g]-2-benzopyran, 1,3,4,6,7,8-hexahydro-4,6,6,7,8,8-hexamethyl- |
| ALICYC | HELVETOLIDE® | 1-Propanol, 2-[1-(3,3-dimethylcyclohexyl)ethoxy]-2-methyl-, 1-propanoat |

| | | |
|---|---|---|
| MACRO = macrocyclische Moschusriechstoffe POLY = polycyclische Mochusriechstoffe ALICYC = alicyclischer Mochusriechstoff | | |

Weiter bevorzugt als Teil des Bestandteils (B) (ii) sind polycyclische und/oder macrocyclische Moschusriechstoffe, wobei sich insbesondere macrocyclische Moschusriechstoffe als besonders vorteilhaft im Sinne der Erfindung gezeigt haben, welche wiederum vorzugsweise gewählt sind aus der Gruppe bestehend aus macrocyclischen C₁₄-C₁₈-Ketonen und macrocyclischen C₁₄-C₁₈-Lactonen, wobei das Keton bzw. Lacton eine Ringgröße von 15 bis 17 Ringatomen und kein, ein oder zwei Sauerstoffatome im Ring aufweist.

Am meisten bevorzugt sind 3-Methylcyclopentadecenon (Muscenon), 15-Pentadec-(11/12)-enolid (Globalide®), Ethylenbrassylat, Oxacyclohexadecan-2-on (Macrolide®), Cyclohexadecanon (Isomuscone®), 8-Cyclohexadecanon (Globanone®), (7/8)-Cyclohexadecanon (Aurelione®) und deren Mischungen.

In einer bevorzugten Ausführungsform wird Bestandteil (B) (ii) gewählt aus der Gruppe bestehend aus 15-Pentadec-(11/12)-enolid (Globalide)®, Ethylenbrassylat und Oxacyclohexadecan-2-on (Macrolide®) und deren Mischungen.

Die Gesamtmenge des oder der (als bevorzugt bezeichneten) Riechstoffe des Bestandteils (B) (ii) (Fondnote) einer erfindungsgemäß bevorzugten Zusammensetzung bzw. eines erfindungsgemäß bevorzugten kosmetischen Produktes beträgt 5 bis 80 Gew.-%, besonders bevorzugt 10 bis 70 Gew.-%, jeweils bezogen auf das Gesamtgewicht des Bestandteils (B), vorzugsweise jeweils bezogen auf das Gesamtgewicht des Bestandteils (B*).

In einer erfindungsgemäßen Zusammensetzung oder einem erfindungsgemäßen kosmetischen Produkt (jeweils insbesondere in einer der als bevorzugt angegebenen Ausgestaltungen) werden vorzugsweise zusätzlich einer oder mehrere Effekte bewirkt, die ausgewählt sind aus der Gruppe von Effekten bestehend aus:
- Reduzierung der Verdampfung von Riechstoffen, insbesondere unmittelbar nach der Applikation der erfindungsgemäßen Zusammensetzung und insbesondere der Kopfnote und/oder der Fondnote; dies gilt auch für (leichtflüchtige) Riechstoffe, die üblicherweise durch die Verdampfung von Lösungsmitteln wie Ethanol/Wasser kurz nach deren Applikation mitgerissen werden;
- zeitliche Stabilisierung des Geruchsprofils, das heisst, dass über einen längeren Zeitraum ein gleichbleibenderes Geruchsprofil erreicht wird;
- Erhöhung oder zeitliche Stabilisierung des geruchlichen Impacts (der wahrgenommenen Geruchsstärke); das heißt, dass der geruchliche Impact (die wahrgenommene Geruchsstärke) über einen langen Zeitraum nicht oder nicht nennenswert verringert wird;
- Verlängerung der Haftung der Riechstoffe des Bestandteils (B) einer erfindungsgemäßen Zusammensetzung, insbesondere der leichtflüchtigen Riechstoffe (Kopfnote) und/oder der Fondnote, dabei wiederum vorzugsweise des Bestandteils (B) (i) und/oder (B) (ii), auf Haut und/oder Haar, insbesondere auf menschlicher Haut, wobei die Retention (Rückhaltung) des Bestandteils (B) einer erfindungsgemäßen Zusammensetzung oder eines erfindungsgemäßen kosmetischen Produktes höher ist im Vergleich zu einer ansonsten identischen Zusammensetzung oder einem ansonsten identischen Produkt, welche(s) keinen Bestandteil (A) enthält;
- Vermittlung eines angenehmen Hautgefühls einer Riechstoffzusammensetzung.

Die erfindungsgemäßen Zusammensetzungen sind vorzugsweise Bestandteil von kosmetischen, insbesondere topischen, kosmetischen Produkten. Bei einer erfindungsgemäßen Zusammensetzung oder einem erfindungsgemäßen kosmetischen (topischen) Produkt enthaltend eine sensorisch (geruchlich) wirksame Menge einer erfindungsgemäßen Zusammensetzung, wird eine Verlängerung der Haftung (Fixierung) der Riechstoffe des Bestandteils (B) bewirkt, bevorzugt auf Haut und/oder Haar, insbesondere auf menschlicher Haut. Dabei werden vorzugsweise ein oder mehrere weitere im Rahmen der vorliegenden Erfindung beschriebenen Effekte erzielt, insbesondere bei den als bevorzugt bezeichneten Ausgestaltungen.

Die verlängerte Haftung (Fixierung) der Riechstoffe des Bestandteils (B) wurde insbesondere bei leichtflüchtigen Riechstoffen (Kopfnote) und/oder der Fondnote beobachtet, dabei wiederum vorzugsweise des Bestandteils (B) (i) und/oder (B) (ii), auf Haut und/oder Haar, insbesondere auf menschlicher Haut, wobei die Retention (Rückhaltung) des Bestandteils (B) einer erfindungsgemäßen Zusammensetzung oder eines erfindungsgemäßen kosmetischen Produktes vorzugsweise mindestens 10 Gew.-%, bevorzugt mindestens 15 Gew.-%, weiter bevorzugt mindestens 20 Gew.-% höher ist im Vergleich zu einer ansonsten identischen Zusammensetzung oder einem ansonsten identischen Produkt, welche(s) keinen Bestandteil (A) enthält.

Hinsichtlich der Fondnote einer erfindungsgemäßen Zusammensetzung oder eines erfindungsgemäßen kosmetischen Produktes, insbesondere hinsichtlich der bevorzugten Riechstoffe des Bestandteils (B) (ii), wird regelmäßig um mindestens 30 Gew.-%, bevorzugt mindestens 40 Gew.-% höhere Retention beobachtet im Vergleich zu einer ansonsten identischen Zusammensetzung oder einem ansonsten identischen Produkt, welche(s) keinen Bestandteil (A) enthält.

Eine erfindungsgemäße Zusammensetzung oder ein (kosmetisches) Produkt (insbesondere in einer der vorstehend als bevorzugt gekennzeichneten Ausgestaltungen) umfasst in einer bevorzugten Ausgestaltung als weiteren Bestandteil eine wirksame Menge
(C) eines oder mehrerer kosmetisch akzeptabler Lösungsvermittler für Bestandteil (B), vorzugsweise für Bestandteil (B*), (B) (i) und/oder (B) (ii), vorzugsweise gewählt aus der Gruppe bestehend aus
   Ethanol
   Dipropylenglycol, Diethylphtalat, Triethylcitrat, Isopropylmyristat und Triacetin, dabei bevorzugt ist Dipropylenglycol.

Lösungsvermittler gemäß Bestandteil (C), insbesondere die als bevorzugt angegebenen kosmetisch akzeptablen Lösungsvermittler, werden im Rahmen dieses Textes nicht als Riechstoffe aufgefasst, insbesondere nicht als Parfümerie-Riechstoffe. Bestandteil (C) ist im Rahmen der vorliegenden Erfindung nicht als Teil des Bestandteils (B) anzusehen und wird diesem folglich nicht zugerechnet.

Bestandteil (C) ist als Lösungsvermittler für Bestandteil (B) vorteilhaft, so dass eine bessere Mischbarkeit und (Ent)Mischungsstabilität von Bestandteil (B) mit Bestandteil (A) gegeben ist, wodurch eine leichtere Handhabung und bessere Weiterverarbeitung ermöglicht wird, was bei der Herstellung von erfindungsgemäßen kosmetischen Produkten von Vorteil ist.

Sofern eine erfindungsgemäße Zusammensetzung bzw. ein erfindungsgemäßes kosmetisches Produkt einen Lösungsvermittler gemäß Bestandteil (C) enthält, der gleichzeitig ein Riechstoff ist, wird dieser, insbesondere für quantitative Betrachtungen, als Bestandteil (B) angesehen und diesem zugeordnet.

Sofern eine erfindungsgemäße Zusammensetzung bzw. ein erfindungsgemäßes kosmetisches Produkt einen Lösungsvermittler gemäß Bestandteil (C) enthält, der gleichzeitig ein Diol- oder Triol gemäß Bestandteil (D) ist, wird dieser, insbesondere für quantitative Betrachtungen, als Bestandteil (D) angesehen und diesem zugeordnet.

Sofern ein erfindungsgemäßes kosmetisches Produkt Bestandteil (C) (i) Ethanol enthält, kann die Gesamtmenge des Bestandteils (C) (i) vorzugsweise bis zu 95 Gew.-% Ethanol, bevorzugt höchstens 90 Gew.-%, betragen, jeweils bezogen auf das Gesamtgewicht des erfindungsgemäßen Produktes.

Eine erfindungsgemäße Zusammensetzung bzw. ein erfindungsgemäßes kosmetisches Produkt enthält Bestandteil (C) (ii) vorzugsweise in einer Gesamtmenge von bis zu 80 Gew.-%, bevorzugt von 0,5 bis 60 Gew.-%, bevorzugt von 1 bis 50 Gew.-%, weiter bevorzugt von 5 bis 40 Gew.-%, jeweils bezogen auf das Gesamtgewicht des Bestandteils (B).

Eine erfindungsgemäße Zusammensetzung (insbesondere in einer der vorstehend als bevorzugt gekennzeichneten Ausgestaltungen) umfasst in einer alternativen bevorzugten Ausgestaltung als zusätzlichen Bestandteil
(D) ein oder mehrere Di- oder Triole mit 3 bis 12 C-Atomen, vorzugsweise gewählt aus der Gruppe bestehend aus Glycerin, 1,2-Propylenglykol, 1,2-Butylenglykol, 1,3-Butylenglykol und Alkandiolen mit 5 bis 12 C-Atomen.

Sofern eine erfindungsgemäße Zusammensetzung bzw. ein erfindungsgemäßes kosmetisches Produkt ein Diol oder Triol gemäß Bestandteil (D) enthält, welches gleichzeitig ein Riechstoff ist, wird dieses, insbesondere für quantitative Betrachtungen, als Bestandteil (D) angesehen und diesem zugeordnet. Bei Bestandteil (D), insbesondere bei den als bevorzugt angegebenen Diolen und Triolen, handelt es sich nicht um Riechstoffe, insbesondere nicht um Parfümerie-Riechstoffe.

Sofern eine erfindungsgemäße Zusammensetzung bzw. ein erfindungsgemäßes kosmetisches Produkt ein Diol- oder Triol gemäß Bestandteil (D) enthält, welches gleichzeitig einen Lösungsvermittler gemäß Bestandteil (C) ist, wird dieses, insbesondere für quantitative Betrachtungen, als Bestandteil (D) angesehen und diesem zugeordnet.

Die Alkandiole mit 5 bis 12 C-Atomen sind dabei vorzugsweise gewählt aus der Gruppe der geradkettigen 1,2-Alkandiole mit 5 bis 10 C-Atomen, insbesondere aus der Gruppe bestehend aus 1,2-Pentandiol, 1,2-Hexandiol, 1,2-Octandiol und/oder 1,2-Decandiol.

Besonders bevorzugte Di- oder Triole des Bestandteils (D) sind gewählt aus der Gruppe bestehend aus Glycerin, 1,2-Propylenglykol, 1,2-Butylenglykol, 1,3-Butylenglykol, 1,2-Pentandiol, 1,2-Hexandiol, 1,2-Octandiol und 1,2-Decandiol.

Bestandteil (D) kann eventuelle unerwünschte Trübungen reduzieren oder verhindern. Zudem kann durch Bestandteil (D) zusätzlich die Haftung einer erfindungsgemäßen Zusammensetzung auf (menschlicher) Haut und/oder (menschlichem) Haar, insbesondere auf menschlicher Haut, und/oder der geruchliche Profilverlauf einer erfindungsgemäßen Zusammensetzung weiter verbessert werden.

Eine erfindungsgemäße kosmetische Zubereitung enthält Bestandteil (D) vorzugsweise in einer Gesamtmenge von 0,2 bis 20 Gew.-%, bevorzugt von 0,5 bis 10 Gew.-%, weiter bevorzugt von 1 bis 5 Gew.-%, jeweils bezogen auf das Gesamtgewicht der kosmetischen Zubereitung.

In einer erfindungsgemäßen Zusammensetzung (insbesondere in einer der vorstehend als bevorzugt angegebenen Ausgestaltungen) liegt Bestandteil (D) vorzugsweise in einer wirksamen Menge vor, das heißt in einer Menge, bei der in der Zusammensetzung durch den Bestandteil (D) einer oder mehrere Effekte bewirkt und/oder verstärkt werden, die ausgewählt sind aus der Gruppe von Effekten bestehend aus:
- weitere Reduzierung der Verdampfung von Riechstoffen, insbesondere unmittelbar nach der Applikation der erfindungsgemäßen Zusammensetzung und insbesondere der Kopfnote und/oder der Fondnote;
- eine weiter verlängerte Haftung auf (menschlicher) Haut und/oder (menschlichem) Haar, insbesondere auf menschlicher Haut (damit ist auch hier menschliche Haut gemeint, bei der es sich nicht um (Mund)Schleimhaut handelt), insbesondere der Topnote und/oder der Fondnote;
- einen über eine längere Zeit weitgehend gleichbleibenden Geruchseindruck nach Applikation auf die (menschliche) Haut;
- einen höheren Impact nach Applikation auf die (menschliche) Haut;
- weitere zeitliche Stabilisierung des Geruchsprofils, das heißt, dass über einen längeren Zeitraum ein noch gleichbleibenderes Geruchsprofil erreicht wird;
- Erhöhung oder weitere zeitliche Stabilisierung des geruchlichen Impacts (der wahrgenommenen Geruchsstärke); abhängig von den jeweiligen eingesetzten Konzentrationen des Bestandteils (D) innerhalb der erfindungsgemäßen Zusammensetzung wird der Impact über einen mehr oder weniger langen Zeitraum erhöht;
- Verlängerung der Haftung der Zusammensetzung, insbesondere der leichtflüchtigen Riechstoffe (Kopfnote), auf Haut und/oder Haar, insbesondere auf menschlicher Haut,
- Erhöhung oder Verlängerung der Diffusivität (Raumwirkung) der Zusammensetzung, insbesondere der leichtflüchtigen Riechstoffe (Kopfnote), auf Haut und/oder Haar, insbesondere auf menschlicher Haut;
- Vermittlung eines weiter verbesserten angenehmen Hautgefühls.

Eine erfindungsgemäße Zusammensetzung hinterlässt vorzugsweise keinen Film auf Haut und/oder Haar und wird somit nicht als störend empfunden. Kurze Zeit nach dem Auftragen (das heißt nach dem Trocknen und ggf. Einziehen) ist eine erfindungsgemäße Zusammensetzung regelmäßig nicht mehr sichtbar (Ausnahme: Haarpflegemittel wie beispielsweise Haarspray, bei denen die Filmbildung gewünscht ist).

Erfindungsgemäße Zusammensetzungen sind demnach - kurz zusammengefasst - Zusammensetzungen mit einer besonders guten Haftung der Kopfnote und/oder Fondnote einer Riechstoffzusammensetzung, einem verlängerten und/oder verstärkten Impact der Kopfnote sowie einem verlängerten Impact der Fondnote, wobei vorzugsweise eine Minderung der Herznote nicht oder zumindest nicht in nennenswerten Umfang zu beobachten ist.

Weitere Stoffe, die Teil einer erfindungsgemäßen (kosmetischen) Zusammensetzung oder einem erfindungsgemäßen kosmetischen (topischen) Produkt sein können, sind:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, weitere Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, (weitere) Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, (weitere) rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-beta-dicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und weitere Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, weitere Riechstoffe, die nicht Teil der Bestandteile (B) (i) und (B) (ii) sind, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten.

Bevorzugte erfindungsgemäße kosmetische Produkte als Ausgestaltungen einer erfindungsgemäßen Zusammensetzung sind gewählt aus der Gruppe bestehend aus: Parfüm-Extraits, Eau de Parfums, Eau de Toilettes, Rasierwässer, Eau de Colognes, Preshave-Produkte, Splash-Colognes und parfümierten Erfrischungstüchern sowie die Parfümierung von sauren, alkalischen und neutralen Reinigungsmitteln, vorzugsweise Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln, vorzugsweise festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ, vorzugsweise Hautcremes- und -lotionen, Gesichtscremes und -lotionen, Tagescremes, Nachtcremes, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten, vorzugsweise Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien, vorzugsweise Achselsprays, Roll-ons, Deosticks, Deocremes, Produkten der dekorativen Kosmetik, vorzugsweise Lidschatten, Nagellacke, Make-ups, Lippenstifte, Mascara sowie von Kerzen, Lampenölen, Räucherstäbchen, Insektiziden und Repellentien.

Bevorzugte erfindungsgemäße Zubereitungen sind kosmetische, insbesondere topische Zubereitungen, die wie üblich zusammengesetzt sind und dem kosmetischen Lichtschutz, zur Behandlung, der Pflege und der Reinigung der Haut und/oder der Haare oder als Schminkprodukt in der dekorativen Kosmetik dienen. Entsprechend liegen derartige Zubereitungen vor als Reinigungsmittel, vorzugsweise als Seife, Syndet, flüssige Wasch-, Dusch-, und Badepräparat, Hautpflegemittel, vorzugsweise als Emulsion (als Lösung, Dispersion, Suspension; Creme, Lotion oder Milch je nach Herstellungsverfahren und Inhaltsstoffen vom Typ Öl-in-Wasser (O/W), vom Typ Wasser-in-Öl (W/O), oder multiple Emulsion, PIT Emulsion, Emulsionsschaum, Mikro-, Nanoemulsion, Pickering Emulsion), Salbe, Paste, Gel (inklusive Hydro-, Hydrodispersions-, Oleogel), alkoholische oder wässrig/alkoholische Lösung, Öl, Toner, Balsam, Serum, Puder, Wipe, Eau de Toilette, Eau de Cologne, Perfum, Wachs, inklusive der Darreichungsform als Stift, Roll-On, (Pump-)Spray, Aerosol (schäumend, nicht schäumend oder nach-schäumend), Hautpflegemittel (wie oben beschrieben) als Fußpflegemittel (inklusive Keratolytika, Desodorant), als Insekten abwehrendes Mittel, als Sonnenschutzmittel, als Selbstbräunungsmittel und /oder Aftersun-Präparat, Hautpflegemittel als Rasiermittel oder After-Shave, als Haarentfernungsmittel, als Haarpflegemittel, vorzugsweise als Shampoo (inklusive Shampoo für normales Haar, für schnellfettendes Haar, für trockenes, strapaziertes (geschädigtes) Haar, 2-in-1 Shampoo, Anti-Schuppen-shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (vorzugsweise Gel oder Wachs); Blondiermittel, Haarfärbemittel, vorzugsweise temporäre, direktziehende, semipermanente Haarfärbemittel, permanente Haarfärbemittel), Hautpflegemittel als dekorative Körperpflegemittel, vorzugsweise Nagelpflegemittel (Nagellack und Nagellackentferner), dekorative Kosmetik (z.B. Puder, Lidschatten, Kajalstift, Lippenstift), Deodorant und / oder Antitranspirant.

### Erfindungsgemäße kosmetische, vorzugsweise topische, Zubereitungen umfassen

Bestandteil (A) und Bestandteil (B), wobei

Bestandteil (A) vorzugsweise in einer Menge von 0,25 - 30 Gew.-%, bevorzugt 0,25 - 20 Gew.-%, weiter bevorzugt 0,5 - 15 Gew.-%, besonders bevorzugt 0,5 - 10 Gew.-%, und ganz besonders bevorzugt 1 - 10 Gew.-%,
und/oder

Bestandteil (B) vorzugsweise in einer Menge von 0,15 - 5 Gew.-%, bevorzugt 0,2 - 3 Gew.-%, weiter bevorzugt 0,3 - 3 Gew.-%, besonders bevorzugt 0,3 - 2,5 Gew.-%, und ganz besonders bevorzugt 0,3 - 2 Gew.-%,
jeweils bezogen auf das Gesamtgewicht der kosmetischen Zubereitung enthalten sind.

Vorzugsweise handelt es sich bei den erfindungsgemäßen Zusammensetzungen um sogenannte "leave-on" - Produkte, d.h. um Produkte, die auf (menschlicher) Haut und/oder (menschlichem) Haar verbleiben und in der Regel nicht abgewaschen werden. Vorzugsweise verbleibt ein erfindungsgemäßes "leave-on" - Produkt (in einer bevorzugten Ausführungsform eines erfindungsgemäßen kosmetischen Produktes) zumindest 15 Minuten oder länger, bevorzugt zumindest zumindest 30 Minuten oder länger und weiter bevorzugt zumindest zumindest 60 Minuten oder länger auf der (menschlichen) Haut und/oder dem (menschlichen) Haar. Hierzu zählen insbesondere Eau de Parfum, Eau de Toilette, Rasierwasser, (Haut)Cremes, Emulsionen (zur topischen Anwendung), Deosprays, Deo-Roller, Haarsprays, Haarconditioner.

Bevorzugte "leave-on" - Produkte sind gewählt aus der Gruppe bestehend aus:
- Hautcreme- oder -lotion, Gesichtscreme oder -lotion, Tagescreme, Nachtcreme, Sonnenschutzcreme, -spray oder -lotion, After-sun-creme oder -lotion, Handcreme oder -lotion, Fußcreme oder -lotion, After-shave-Creme oder -lotion, Hautaufhellungscreme oder -lotion, Hautbräunungscreme oder -lotion;
- Haarpflegeprodukte, vorzugsweise Haarspray, Haargel, festigenden Haarlotion, Haarwasser, Haarcreme, Haarwachs, Haarlotion, Haarconditioner;
- Deodorantien und Antiperspirantien, vorzugsweise Achselspray, Roll-on, Deostick, Deocreme, und
- Produkte der dekorativen Kosmetik, vorzugsweise Lidschatten, Nagellack, (pflegender) Lippenstift oder Mascara.

Bevorzugt ist eine erfindungsgemäße Zusammensetzung oder ein erfindungsgemäßes kosmetisches Produkt, vorzugsweise in einer der vorstehend als bevorzugt angegebenen Ausgestaltungen, umfassend die Bestandteile (A) und (B), enthaltend
(A) Cetylnonanoat und/oder Stearylnonanoat in einer Gesamtmenge von 0,25 - 30 Gew.-%, bevorzugt 0,25 - 20 Gew.-%, weiter bevorzugt 0,5 - 15 Gew.-%, besonders bevorzugt 0,5 - 10 Gew.-%, und ganz besonders bevorzugt 1 - 10 Gew.-%,
   und/oder
(B) einen oder mehrere Riechstoffe in einer Menge von 0,15 - 5 Gew.-%, bevorzugt 0,2 - 3 Gew.-%, weiter bevorzugt 0,3 - 3 Gew.-%, besonders bevorzugt 0,3 - 2,5 Gew.-%, und ganz besonders bevorzugt 0,3 - 2 Gew.-%, wobei vorzugsweise Bestandteil (B)
   (B) (i) einen, 2, 3, 4, 5 oder mehr Riechstoffe mit einem Molgewicht im Bereich von 100 g/mol bis 175 g/mol (Topnote) enthält, vorzugsweise mit einem Molgewicht im Bereich von 110 g/mol bis 160 g/mol, bevorzugt im Bereich von 115 g/mol bis 160 g/mol, besonders bevorzugt im Bereich von 120 g/mol bis 155 g/mol,
   und/oder
   (B) (ii) einen, 2, 3, 4, 5 oder mehr Riechstoffe mit einem Molgewicht von größer oder gleich 190 g/mol (Fondnote) enthält, bevorzugt im Bereich von 190 g/mol bis 300 g/mol, weiter bevorzugt mit einem Molgewicht im Bereich von 195 g/mol bis 290 g/mol, und am meisten bevorzugt im Bereich 200 bis 275 g/mol,
   und/oder
   (C) (ii) Dipropylenglycol, Diethylphtalat, Triethylcitrat, Isopropylmyristat und Triacetin, wobei die Gesamtmenge des Bestandteils (C) (ii) bis zu 80 Gew.-%, bevorzugt von 0,5 bis 60 Gew.-%, bevorzugt von 1 bis 50 Gew.-%, weiter bevorzugt von 5 bis 40 Gew.-%, jeweils bezogen auf das Gesamtgewicht des Bestandteils (B) beträgt,
   und/oder
(D) ein oder mehrere Di- oder Triole mit 3 bis 12 C-Atomen, vorzugsweise gewählt aus der Gruppe bestehend aus Glycerin, 1,2-Propylenglykol, 1,2-Butylenglykol, 1,3-Butylenglykol, 1,2-Pentandiol, 1,2-Hexandiol, 1,2-Octandiol und 1,2-Decandiol, in einer Gesamtmenge von 0,2 bis 20 Gew.-%, bevorzugt von 0,5 bis 10 Gew.-%, weiter bevorzugt von 1 bis 5 Gew.-%,
jeweils bezogen auf das Gesamtgewicht der Zusammensetzung bzw. des Produktes.

Die Gewichtsprozentangaben sind jeweils bezogen auf das Gesamtgewicht der Zusammensetzung. Vorzugsweise sind die zu den Bestandteilen (A), (B), (C) und (D) genannten bevorzugten Gewichtsanteile gleichzeitig eingestellt.

Die vorliegende Erfindung betrifft auch die Verwendung des Bestandteils (A) (wie oben definiert, vorzugsweise in einer oben als besonders bevorzugt angegebenen Ausführungsform) als Mittel zur
- Reduzierung der Verdampfung von Riechstoffen in einer Riechstoffzusammensetzung, insbesondere unmittelbar nach der Applikation;
- zeitlichen Stabilisierung des Geruchsprofils einer Riechstoffzusammensetzung;
- Erhöhung oder zeitlichen Stabilisierung des geruchlichen Impacts (der wahrgenommenen Geruchsstärke) einer Riechstoffzusammensetzung;
- Verlängerung der Haftung von Riechstoffen einer Riechstoffzusammensetzung, insbesondere der Kopfnote und/oder Fondnote, auf Haut und/oder Haar, insbesondere auf menschlicher Haut;
   und/oder
- Vermittlung oder Verstärkung eines angenehmen Hautgefühls einer Riechstoffzusammensetzung.

Die vorliegende Erfindung betrifft ferner ein Verfahren zur
- Reduzierung der Verdampfung von Riechstoffen in einer Riechstoffzusammensetzung, insbesondere unmittelbar nach der Applikation,
- zeitlichen Stabilisierung des Geruchsprofils einer Riechstoffzusammensetzung,
- Erhöhung oder zeitlichen Stabilisierung des geruchlichen Impacts (der wahrgenommenen Geruchsstärke) einer Riechstoffzusammensetzung,
- Verlängerung der Haftung von Riechstoffen einer Riechstoffzusammensetzung, insbesondere der Kopfnote und/oder Fondnote, auf Haut und/oder Haar, insbesondere auf menschlicher Haut,
   und/oder
- Vermittlung oder Verstärkung eines angenehmen Hautgefühls einer Riechstoffzusammensetzung,
   mit folgendem Schritt:

Mischen von
   (A) einer Bestandteil (B) fixierenden Menge an Cetylnonanoat und/oder Stearylnonanoat
      und
   (B) einem oder mehreren Riechstoffen,
   wobei vorzugsweise das Massenverhältnis des Bestandteils (A) zur Gesamtmasse des Bestandteils (B) im Bereich von 1 : 20 bis 200 : 1, bevorzugt im Bereich von 1 : 12 bis 100 : 1, liegt, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1, liegt,
   sowie gegebenenfalls weiteren Bestandteilen, vorzugsweise gewählt aus der Gruppe bestehend aus den (oben bevorzugt genannten) Bestandteilen (C) und/oder (D),
   wobei das Massenverhältnis bezogen ist auf das Gesamtgewicht der resultierenden Zusammensetzung nach dem Mischen.

Die erfindungsgemäßen Zusammensetzungen lassen sich auf einfache Weise herstellen durch Mischen der Einzelkomponenten der Bestandteile (A) und (B) sowie gegebenenfalls (C) und/oder (D). Dabei ist die Reihenfolge der Kontaktierung der Einzelkomponenten beziehungsweise Bestandteile nicht kritisch und kann variiert werden.

Für die erfindungsgemäßen Verfahren gelten die obigen Angaben zu bevorzugten erfindungsgemäßen Zusammensetzungen und kosmetischen Produkten entsprechend.

Die vorliegende Erfindung betrifft auch ein Verfahren zur
- Vermittlung, Verstärkung oder Modifizierung eines Geruchs auf (menschlicher) Haut,
- Verlängerung der Haftung von Riechstoffen einer Riechstoffzusammensetzung, insbesondere der Kopfnote und/oder Fondnote, auf Haut und/oder Haar, insbesondere auf menschlicher Haut,
   und/oder
- Vermittlung oder Verstärkung eines angenehmen Hautgefühls einer Riechstoffzusammensetzung,
   mit folgendem Schritt:
- Applizieren einer erfindungsgemäßen Zusammensetzung (bevorzugt einer Zusammensetzung, die oben als bevorzugt angegeben ist) auf die (menschliche) Haut.

Ferner hat sich gezeigt, dass Bestandteil (A) sich nicht nur wie oben bereits beschrieben als Fixateur für Riechstoffe eignet, sondern dass Bestandteil (A) ebenfalls als Lösemittel für kosmetische UV-Lichtschutzfilter, insbesondere für lipophile (kristalline) UV-Lichtschutzfilter. Daneben wurde gefunden, dass Bestandteil (A) aufgrund der gleichmäßigen Verteilung der (vorzugsweise lipophilen) kosmetischen UV-Filter eine SPF-Erhöhung bewirkt, d.h. einen höheren Lichtschutzfaktor und damit eine höhere UV-Schutzleistung. Geeignete und bevorzugte UV-Filter sind dabei die in WO 2005/123101 genannten.

Darüberhinaus wurde gefunden, dass Bestandteil (A) den Glanz von menschlichem und tierischem Haar verbessert. Insoweit sind gemäß eines Aspekts der vorliegenden Erfindung Haarpflegemittel bevorzugt, vorzugsweise gewählt aus der Gruppe bestehend aus Shampoo, dabei wiederum bevorzugt sind Shampoo für normales Haar, für schnellfettendes Haar, für trockenes, strapaziertes (geschädigtes) Haar, 2-in-1 Shampoo, Anti-Schuppen-shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (vorzugsweise Gel oder Wachs); Haarfärbemittel.

Darüberhinaus wurde gefunden, dass Bestandteil (A) hautfeuchteregulierend wirkt, indem der Hautfeuchtegehalt (menschlicher) Haut verbessert und der transepidermale Wasserverlust (TEWL) reduziert wird, ohne dabei okkludierende Eigenschaften zu besitzen.

### Weitere Eigenschaften des Bestandteils (A) in erfindungsgemäßen Zusammensetzungen und erfindungsgemäßen kosmetischen Produkten:

- mittlere Hautabsorption
- bei Raumtemperatur (ca. 20°C) fest
- gutes (kosmetisches) Feuchthaltemittel (moisturizer)
- sehr gute Rückfettungseigenschaften
- geringe Polarität
- merkbar verbessertes Hautgefühl
- bietet einen Wasser abweisenden Effekt
- hochbeständig gegen Oxidation
- schützt die (menschliche) Haut vor dem Austrocknen
- nicht okklusiv
- gibt ein weiches, glattes und geschmeidiges Hautgefühl
- bewirkt gute Rückfettung, z.B. als Bestandteil von Deodorantien, Lippenstiften oder Shampoos
- gibt Emulsionen Konsistenz und verbessert deren Stabilität.

Die folgenden Beispiele erläutern die Erfindung; sofern nicht anders angegeben beziehen sich Anteile und Prozente auf das Gewicht.

### Beispiele

### Verwendete Abkürzungen: TEA = Triethanolamin, SPF = Lichtschutzfaktor (sun protection factor); MW = Molgewicht; Ret. = Retention; DPG = Dipropylenglycol

In sämtlichen folgenden Beispielen wurde das im Folgenden als "Mischung S" bezeichnete Gemisch enthaltend Cetylnonanoat und Stearylnonanoat verwendet, welches folgendemaßen zusammengesetzt war: 67,6 Gew.-% Cetylnonanoat, 27,8 Gew.-% Stearylnonanoat, 2,1 Gew.-% Cetyl-2-methyloctanoat, 1,3 Gew.-% Myristyl nonanoat, 0,9% Stearyl-2-methyloctanoat.

### Test 1: Untersuchung der reduzierten Freisetzung von Riechstoffen aus einem Riechstoffgemisch enthaltend 10 Gew.-% an "Mischung S"

### Testdurchführung:

50 µl eines Riechstoffgemisches enthaltend die in der nachfolgenden Tabelle aufgelisteten Riechstoffe wurden zu 0,5 % in EtOH gelöst. Einem Aliquot der ethanolischen Lösung wurden 10 Gew.-% (bezogen auf die Menge an eingesetztem Parfümöl) eines Gemisches enthaltend Cetylnonanoat und Stearylnonanoat zugesetzt.

Die beiden Lösungen wurden jeweils auf eine definierte Fläche Riechstreifen (2 cm²) aufgetragen und 15 min bei 22 °C äqulibriert. Anschließend wurden die Riechstreifen mit jeweils 4 ml Aceton extrahiert und mit 100 µg Diphenylmethan als Internen Standard (IS) versetzt. Die Proben wurden im Anschluss per GC/MS vermessen, die auf den Riechstoffen verbliebene Menge an Riechstoffen mittels IS-Methode quantifiziert.

Die nachfolgende Tabelle zeigt die Ergebnisse der durchgeführten quantitativen Analysen und die daraus berechnete prozentuale Erhöhung der Retention des jeweiligen Parfümerie-Riechstoffes.

| **Riechstoff** | **MW** | **GC-Counts** **mit (A)** | **GC-Counts** **ohne (A)** | **Ret. %** | **Bestandteil** | |
|---|---|---|---|---|---|---|
| Melonal | 140,23 | 71121 | 50312 | **41** | (B) (i) | |
| n-Heptanol | 118,23 | 133295 | 65987 | **102** | (B) (i) | |
| Dihydromyrcenol | 156,27 | 329964 | 250262 | **32** | (B) (i) | |
| Campher | 152,24 | 532089 | 446670 | **19** | (B) (i) | |
| Methylbenzoat | 136,15 | 85111 | 39451 | **116** | (B) (i) | |
| Agrunitril | 151,25 | 249678 | 95046 | **163** | (B) (i) | |
| Geraniol | 154,25 | 359840 | 211026 | **71** | (B) (i) | |
| 2-Phenylethylalkohol | 122,17 | 408413 | 317311 | **29** | (B) (i) | |
| Linalool | 154,25 | 213631 | 121770 | **75** | (B) (i) | |
| Anisaldehyd | 136,15 | 440213 | 291215 | **51** | (B) (i) | |
| Vanillin | 152,15 | 251478 | 177240 | **42** | (B) (i) | |
| Isobornylacetat | 196,29 | 259575 | 75344 | **245** | | (B) (ii) |
| α-Damascon | 192,30 | 384948 | 113119 | **240** | | (B) (ii) |
| β-Ionon | 192,30 | 514323 | 266958 | **93** | | (B) (ii) |
| Lilial | 204,31 | 610208 | 346963 | **76** | | (B) (ii) |
| Cedrol | 222,40 | 812898 | 518214 | **57** | | (B) (ii) |
| γ-Dodecalacton | 198,30 | 615353 | 326625 | **88** | | (B) (ii) |
| Hexylsalicylat | 222,28 | 794870 | 477405 | **66** | | (B) (ii) |
| Helional | 192,22 | 510281 | 328197 | **55** | | (B) (ii) |
| Benzylbenzoat | 212,25 | 843218 | 578062 | **46** | | (B) (ii) |
| ω-Hexadecanolid | 254,40 | 619509 | 421691 | **47** | | (B) (ii) |
| Ethylenbrassylat | 270,37 | 651864 | 428545 | **52** | | (B) (ii) |

| | | | | | | |
|---|---|---|---|---|---|---|
| Melonal = 2,6-Dimethyl-5-hepten-1-al; Helional = 2-Methyl-3-(3,4-methylendioxyphenyl)propanal; Agrunitril = 3,7-Dimethyl-6-octen-1-nitril; Lilial = 2-Methyl-3-(4-tert-butylphenyl)propanal. | | | | | | |

Die Ergebnisse der quantitativen Untersuchungen belegen eindeutig die fixierenden Eigenschaften von Cetylnonanoat und Stearylnonanoat. In Abhängigkeit vom jeweiligen Riechstoff konnten höhere Mengen in einem prozentualen Bereich von 19% (Campher) bis 240% (alpha-Damascon) nachgewiesen werden. Dies zeigt deutlich, dass die Abdampfrate von Riechstoffen mit sehr unterschiedlicher chemischer Struktur in Anwesenheit eines Gemisches enthaltend Cetylnonanoat und Stearylnonanoat signifikant reduziert werden kann, wodurch die fixierenden Eigenschaften dieser Ester für Riechstoffe eindeutig belegt werden konnte. Diese analytischen Ergebnisse konnten sensorisch nachvollzogen werden.

**Formulierungsbeispiele kosmetischer Produkte enthaltend eine erfindungsgemäße Zusammensetzung**

| | |
|---|---|
| 1 = Deostift | 7 = Sonnenschutz-Stick mit SPF 50 |
| 2 = Creme-Duschgel | 8 = Haarwachs |
| 3= Deo-Pumpspray | 9 = Sonnenschutzcreme |
| 4 = Lippenpflegecreme mit SPF 30 | 10 = After Sun Spray |
| 5 = Hand- & Körperlotion | 11 = Tagescreme |
| 6 = Feuchtigkeitscreme O/W | 12 = Nachtcreme W/O |

Die jeweilige Zusammensetzung der in den nachfolgenden kosmetischen Produkten 1 bis 12 verwendeten Parfümöle P1 bzw. P2 ist weiter unten beschrieben.

| | | | | | | | | | | | | | |
|---|---|---|---|---|---|---|---|---|---|---|---|---|---|
| | | | | | | | | | | | | | |
| | | | | | | | | | | | | | |

| **"Mischung S"** | | **5,0** | **10,0** | **0,5** | **1,0** | **2,5** | **3,0** | **9,0** | **0,5** | **1,5** | **4,0** | **1,0** | **7,0** |
|---|---|---|---|---|---|---|---|---|---|---|---|---|---|
| (-) alpha Bisabolol, natürlich | Bisabolol | | | | | 0,1 | 0,1 | 0,1 | | | | 0,2 | |
| Abil 350 | Dimethicone | | | | | | 2,0 | | | | | | |
| Aerosil® 200 | Silica | 1,5 | | | | | | | | | | | |
| Allantoin Aluminiumstearat | Allantoin Aluminium Stearate | | | | | | | | | | 0,1 | | |
| Aloe Vera Gel Konzentrat 10/1 | Aloe Barbadensis Leaf Juice | | | | | | 1,0 | | | | | | |
| Avocadoöl | Persea Gratissima (Avocado) Oil | | | | | | | | | | 3,0 | | |
| Bienenwachs | Cera Alba | | | | 3,0 | | | | | | | | |
| Tert.Butyl Hydroxytoluol | BHT | | 0,1 | | | | | | | | | | |
| Biotive® L-Arginin | Arginine | | | | | | | | | 0,5 | | | |
| Carbopol ETD 2050 | Carbomer | | | | | | | | | 0,3 | | | |
| Carbopol Ultrez-10 | Carbomer | | | | | | 0,2 | | | | | 0,2 | |
| Carbopol Ultrez-21 | Acrylates/C10-30 Alkyl Acrylate Crosspolymer | | | | | 0,2 | | | | | | | |
| Rizinusöl | Ricinus Communis (Castor) Seed Oil | | 8,0 | | | | | | | | | | |
| Citronensäure 10% in Wasser | Citric Acid | | 0,2 | | | | | | | | | | |
| Corapan TQ | Diethylhexyl 2,6-Naphthalate | | | | 2,0 | | | | | | | | |
| Kosmetikfarbe, Pulver | Color | | | | | | | | | | | 4,0 | |
| Covi-Ox T-70 | Tocopherol | | | | | | | | | | 0,1 | | |
| Cutina GMS V | Glyceryl Stearate | | | | | 1,0 | 2,0 | | | | | 2,5 | |
| DC 9701 Pulver | Dimethicone/Vinyl Dimethicone Crosspolymer | | | | | | | 2,0 | | | | | |
| Deolite | Dimethyl Phenylpropanol Pentylene Glycol | | | 0,5 | | | | | | | | | |
| Diammoniumcitrat | Diammonium Citrate | | 0,1 | | | | | | | | | | |
| Dow Corning 246 fluid | Cyclohexasiloxane | | | | | | | | | | 2,0 | | |
| Dow Corning 345 fluid | Cyclomethicone | Ad 100 | | | | 0,5 | | | | | | | |
| D-Panthenol 75 L | Panthenol | | 0,5 | | | | | | | | 1,0 | | |
| Dracorin® 100 S.E.P. | Glyceryl Stearate, PEG-100 Stearate | 1,0 | | | | | | | | | | | |
| Dracorin® CE | Glyceryl Stearate/Citrate | | | | | | | | | 2,0 | | | |
| Dracorin® GOC | Glyceryl Oleate Citrate Caprylic Capric Triglyceride | | | | | 2,0 | | 0,5 | | | 2,0 | | |
| Drago-Beta-Glucan | Water (Aqua), Butylene Glycol, Glycerin, Avena Sativa (Oat) Kernel Extract | | | | | 1,5 | | | | | | 2,0 | |
| DragoCalm® | Water, Glycerin, Avena Sativa (Oat Kernel Extract) | | | | 1,0 | | | | | | | | |
| Dragocide® Liquid | Phenoxyethanol, Methylparaben, Ethylparaben, Butylparaben, Propylparaben, Isobutylparaben | | | | 0,8 | | | | | 0,8 | | 0,8 | 0,8 |
| Dragoderm® | Glycerin, Triticum Vulgare (Wheat) Gluten, Water (Aqua) | | | | | | | | | | | 2,0 | |
| Dragosan W/O P | Sorbitan Isostearate, Hydrogenated Castor Oil, Ceresin, Beeswax (Cera alba) | | | | | | | | | | | | 8,0 |
| Dragosantol® 100 | Bisabolol | | 0,2 | | 0,1 | | | | | | | | 0,2 |
| Dragosine® | Carnosine | | | | | | | | | | | 0,2 | |
| Dragoxat® 89 | Ethylhexyl Isononanoate | | | | | 3,0 | 4,0 | 5,0 | | 3,0 | | 4,0 | 5,0 |
| EDTA BD | Disodium EDTA | | | | | | | | | 0,1 | | 0,1 | |
| Emulsiphos® | Potassium Cetyl Phosphate, Hydrogenated Palm Glycerides | | | | 2,5 | | 2,0 | | | | | 2,0 | |
| Ätherisches Öl | Essential Oil | | 3,0 | | | | | | | | | | |
| Methanol | Ethanol | | | Ad 100 | | | | | | | 5,0 | | |
| Eusolex T-AVO | Titanium Dioxide, Silica | | | | | | | 5,0 | | | | | |
| Farbstoffe E172 + E171 | Color | | | | | | | 3,0 | | | | | |
| Parfümöl "P1" | Perfume | 1,0 | 2,0 | 3,0 | 0,2 | 0,4 | 0,3 | | | | | | |
| Parfümöl "P2" | Perfume | | | | | | | 0,3 | 0,2 | 0,3 | 0,2 | 0,1 | 0,3 |
| Frescolat® ML | Menthyl Lactate | | | | | | | | | | 0,5 | | |
| Fruitapone® Orange B | Propylene Glycol, Water (Aqua), Citric Acid, Citrus Aurantium Dulcis (Orange) Juice, Trideceth-9, Bisabolol | | | | | | | | | | 1,0 | | |
| Glycerin 85% in Wasser | Glycerin | | | | | 3,0 | 2,0 | | | | | 3,0 | 3,0 |
| Glycerin | Glycerin | | | | 4,0 | | | | | | 4,0 | | |
| Hydrolite®-5 | Pentylene Glycol | | | | | 5,0 | 2,0 | | | | 5,0 | | |
| Hydroviton®-24 | Water, Pentylene Glycol, Glycerin, Lactic Acid, Sodium Lactate, Serine, Urea, Sorbitol, Sodium Chloride, Allantoin | | | | | | 1,0 | | | | | | 2,0 |
| Iso Adipat | Diisopropyl Adipate | | | 0,3 | | | | | | | | | |
| Isodragol® | Triisononanoin | | | | 3,0 | 4,0 | 2,0 | | | | | | |
| Isopropylpalmitat | Isopropyl Palmitate | | | | | | | 13,0 | | | | | |
| Jojoba Öl | Simmondsia Chinensis (Jojoba) Seed Oil | | | | | | | | | | | | 2,0 |
| Keltrol CG RD | Xanthan Gum | | | | 0,4 | 0,1 | 0,1 | | | 0,1 | | 0,1 | |
| Lanette 18 | Stearyl Alcohol | 20,0 | | | | | | | | | | | |
| Lanette E | Sodium Cetearyl Sulfate | | | | 0,75 | | | | | | | | |
| Lanette O | Cetearyl Alcohol | | | | 2,5 | 1,5 | 3,0 | 5,0 | | 2,0 | | 3,0 | |
| Lipex Cocoasoft | Theobroma Cocoa Seed Butter | | | | 2,0 | | | | | | | | |
| Mineralöl | Mineral Oil | | | | | | | | 10,5 | | | | 8,0 |
| Neo Heliopan® 303 | Octocrylene | | | | 7,5 | | | 10,0 | | 5,0 | | | |
| Neo Heliopan® 357 | Butylmethoxydibenzoylmethane | | | | | | | 5,0 | | 4,0 | | 1,5 | |
| Neo Heliopan® AP, 22% in Wasser, neutralisiert mit TEA | Disodium Phenyl Dibenzimidazole Sulfonic Acid | | | | 1,4 | | | | | | | | |
| Neo Heliopan® E 1000 | Isoamyl p.Methoxycinnamate | | | | 7,5 | | | | | | | 5,0 | |
| Neo Heliopan® Hydro, 25% in Wasser, neutralisiert mit L-Arginin | Phenylbenzimidazole Sulfonic Acid | | | | | | | | | 8,0 | | | |
| Neo Heliopan® Hydro, 30% in Waser, neutralisiert mit TEA | Phenylbenzimidazole Sulfonic Acid | | | | 6,7 | | | | | | | 3,3 | |
| Neo Heliopan® OS | Ethylhexyl Salicylate | | | | 5,0 | | | | | 5,0 | | | |
| Neutralöl | Caprylic/Capric Triglyceride | | | | | | | Ad 100 | | | 5,0 | | |
| Ozokerit Wachs 2389 | Ozokerite | | | | | | | | 21,5 | | | | 2,0 |
| PCL Liquid® 100 | Cetearyl Ethylhexanoate | | | | | 7,0 | 5,0 | | 0,5 | | 4,0 | 5,0 | |
| Pemulen TR-2 | Acrylates/C10-30 Alkyl | | | | | | | | | | 0,3 | | |
| | Acrylate Crosspolymer | | | | | | | | | | | | |
| Pluronic® L-31 | Polaxamer 101 | | 3,0 | | | | | | | | | | |
| Polyglycol 1000 | PEG-20 | 5,0 | | | | | | | | | | | |
| Kaliumsorbat | Potassium Sorbate | | | | | 0,1 | | | | | 0,1 | | |
| Propylenglycol | Propylene Glycol | | | 2,0 | | | 3,0 | | | 4,0 | | | |
| Rezal 36 GP | Aluminium Zirconium Tetrachlorohydrex GLY | 10,0 | | | | | | | | | | | |
| Natriumchlorid | Sodium Chloride | | | | | | | | | | | | 1,0 |
| NaOH, 10%ige wässrige Lösung | Sodium Hydroxide | | | | | 0,5 | 0,6 | | | | | | 0,4 |
| Softisan 100 | Hydrogenated Coco Glycerides | | | | | | | | | 1,5 | | | |
| Lösungsverbesserer | PEG-40Hydrogenated Castor Oil, Trideceth-9, Propylene Glycol, Water (Aqua) | | | 2,0 | | | | | | | | | |
| Sojaöl | Glycine Soja (Soybean) Oil | | Ad 100 | | | | | | | | | | |
| Squalan, pflanzlich | Squalane | | | | | | | | | | | 3,0 | |
| Super Hartolan | Lanolin Alcohol | | | | | | | | 0,5 | | | | |
| SymCalmin® | Pentylene Glycol, Butylene Glycol, Hydroxyphenyl Propamidobenzoic Acid | | | | | | 1,0 | | | | | | |
| SymClariol® | Decylene Glycol | 0,3 | | | | | | | | | | | |
| SymDeo® MPP | Dimethyl Phenylbutanol | 0,5 | | | | | | | | | | | |
| SymDiol® 68 | 1,2 Hexanediol, Caprylyl Glycol | | | | | | 0,5 | | | | | | |
| SymGlucan® | Water (Aqua) Glycerin, Beta Glucan | | | | | | | | | | 1,0 | | |
| SymMollient® W/S | Trideceth-9, PEG-5 Isononanoate | | 1,0 | 0,5 | | | | | | | | | |
| SymRelief® | Bisabolol, Zingiber Officinale (Ginger) Root Extract | | | | | | | | | | 0,2 | | |
| SymRepair® | Hexyldecanol, Bisabolol, Cetylhydroxyproline Palmitamide, Stearic Acid, Brassica Campestris (Rapeseed Sterols) | | | | | | 2,0 | | | | 4,0 | | |
| SymVital™ | Aloe Barbadensis Leaf | | | | | 0,1 | | | | | 0,2 | | |
| | Juice Powder, Magnesium Ascorbyl Phosphate, Rubus Idaeus (Raspberry) Leaf Extract | | | | | | | | | | | | |
| Talkum | Talc | 1,0 | | | | | | | | | | | |
| TeCE-Ozokerit N502 | Ozokerite | | | | | | | 23,0 | | | | | |
| Tinosorb S® | Bis-Ethylhexyloxyphenol Methoxyphenyl Triazine | | | | | | | 3,0 | | | | | |
| Triethanolamin (TEA) | Triethanolamine | | | | 0,4 | | | | | | 0,3 | 0,9 | |
| Vaseline | Petrolatum | | | | | | | | Ad 100 | | | | |
| Vitamin E acetat | Tocopherol Acetate | | | | 0,2 | | | 0,7 | | 0,5 | | | 0,2 |
| Wacker Belsil, CDM 3526 VP | C26-C28Alkyl Dimethicone | | | | | | | 2,0 | | | | | |
| Wasser | Water (Aqua) | | | 10,0 | Ad 100 | Ad 100 | Ad 100 | | | Ad 100 | Ad 100 | Ad 100 | Ad 100 |
| Zetesol 100 | MIPA-Laureth Sulfate, Laureth-4, Cocamide DEA | | 43,0 | | | | | | | | | | |
| ZinkOxid | Zinc Oxide | | | | 5,0 | | | | | | | | |

Das in den Formulierungsbeispielen 1 bis 6 eingesetzte Parfümöl "P1" mit Rosengeruch hatte folgende Zusammensetzung:

| Komponente / NAME | Gew.-teile |
|---|---|
| Acetophenon, 10%ig in DPG | 10,00 |
| n-Undecanal | 5,00 |
| Gamma-Undecalacton | 15,00 |
| Allylamylglycolat, 10%ig in DPG | 20,00 |
| Amylsalicylat | 25,00 |
| Benzylacetat | 60,00 |
| Citronellol | 80,00 |
| D-Limonen | 50,00 |
| Decenol trans-9 | 15,00 |
| Dihydromyrcenol | 50,00 |
| Dimethylbenzylcarbinylacetat | 30,00 |
| Diphenyloxid | 5,00 |
| GALAXOLIDE® | 20,00 |
| Geraniol | 40,00 |
| Nerol | 20,00 |
| Geraniumöl | 15,00 |
| Hexenol cis-3, 10%ig in DPG | 5,00 |
| Hexenylsalicylat cis-3 | 20,00 |
| Indol, 10%ig in DPG | 10,00 |
| Alpha-Ionon | 15,00 |
| Beta-Ionon | 5,00 |
| Lilial® (2-Methyl-3-(4-tert-butyl-phenyl)propanal) | 60,00 |
| Linalool | 40,00 |
| Methylphenylacetat | 10,00 |
| Phenylethylalkohol | 245,00 |
| Styrolylacetat | 20,00 |
| Terpineol | 30,00 |
| Tetrahydrolinalool | 50,00 |
| Ethylenbrassylat | 30,00 |
| Summe: | 1.000,00 |

Das in den Formulierungsbeispielen 7 bis 12 eingesetzte Parfümöl "P2" mit einem weißen Blütenduft und Moschusnote hatte folgende Zusammensetzung:

| Komponente / NAME | Gew.-teile |
|---|---|
| Benzylacetat | 60,00 |
| Citronellylacetat | 60,00 |
| Cyclamenaldehyd (2-Methyl-3-(4-isopropylphenyl)propanal | 20,00 |
| Dipropylenglycol | 60,00 |
| Ethyllinalool | 40,00 |
| Florol (2-Isobutyl-4-methyltetrahydro-2*H*-pyran-4-ol) | 30,00 |
| Globanone [(E/Z)-8-Cyclohexadecen-1-on] | 100,00 |
| Ethylenbrassylat | 80,00 |
| Hedione (Methyldihydrojasmonat) | 140,00 |
| Hexenylsalicylat, cis-3 | 10,00 |
| Vertocitral (2,4-dimethyl-3-cyclohexencarboxaldehyd) | 5,00 |
| Hydratropaaldehyd, 10%ig in DPG | 5,00 |
| Isodamascon (1-(2,4,4-Trimethyl-2-cyclohexen-1-yl)-2-buten-1-on), 10%ig in DPG | 5,00 |
| Cyclohexadecanon | 40,00 |
| Jacinthaflor (2-Methyl-4-phenyl-1,3-dioxolan) | 10,00 |
| Cis-Jasmon, 10%ig in DPG | 20,00 |
| Linalool | 50,00 |
| Linalylacetat | 30,00 |
| Methylbenzoat, 10%ig in DPG | 25,00 |
| para-Methylkresol, 10%ig in DPG | 10,00 |
| Nerol | 20,00 |
| Phenylpropylaldehyd | 5,00 |
| 2-Phenylethylalkohol | 82,00 |
| Tetrahydrogeraniol | 13,00 |
| 2,2-Dimethyl-3-cyclohexyl-1-propanol | 40,00 |
| Tonalide® | 40,00 |
| Gesamt: | 1.000,00 |

## Patentansprüche

1. Zusammensetzung umfassend oder bestehend aus
(A) Cetylnonanoat und/oder Stearylnonanoat,
und
(B) einem oder mehreren Riechstoffen, vorzugsweise einer Riechstoffmischung
umfassend 2, 3, 4, 5, 6, 7, 8, 9, 10 oder mehr Riechstoffe,
wobei Bestandteil (A) in einer für den, mehrere oder sämtliche Riechstoffe des Bestandteils (B) fixierenden Menge enthalten ist,
wobei vorzugsweise das Massenverhältnis des Bestandteils (A) zur Gesamtmasse des Bestandteils (B) im Bereich von 1 : 20 bis 200 : 1 liegt, bevorzugt im Bereich von 1 : 12 bis 100 : 1, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1,
mit der Maßgabe, dass die Zusammensetzung kein Drüsensekret von *Trichiocampus grandis* ist bestehend aus 7% Hexadecylpentanoat, 2% Octyloctanoat, 1 % Hexadecylnonanoat, 1 % Octyldecanoat, 3% Hexylhexadecanoat, 24% 2-Hexenylhexadecanoat, 14% 2-Octenylhexadecanoat, 4% Methylicosenoat, 3% Methyldocosanoat, 13% Methyldocosenoat, 2% Methyltetracosanoat, 2% Methyltetracosenoat 1 % Hexadecanal, 2% Tetracosanal, 11% eines Acetogenins, 5% Verunreingung, sowie Spuren von Pentacosan, Heptacosan, Nonacosan, Methylhexadecanoat, Isopropylhexadecanoat, Hexenylhexadecanoat, Heptenylhexadecanoat, Methyloctadecanoat, Methyloctadecenoat, Isopropyloctadecanoat, (Z)-3-Octenal und Octadecanal.

2. Zusammensetzung nach Anspruch 1, **dadurch gekennzeichnet, dass** Bestandteil (B)
(B) (i) einen, 2, 3, 4, 5 oder mehr Riechstoffe mit einem Molgewicht im Bereich von 100 g/mol bis 175 g/mol (Topnote) enthält, vorzugsweise mit einem Molgewicht im Bereich von 110 g/mol bis 160 g/mol, bevorzugt im Bereich von 115 g/mol bis 160 g/mol, besonders bevorzugt im Bereich von 120 g/mol bis 155 g/mol,
und/oder
(B) (ii)einen, 2, 3, 4, 5 oder mehr Riechstoffe mit einem Molgewicht von größer oder gleich 190 g/mol (Fondnote) enthält, vorzugsweise mit einem Molgewicht im Bereich von 190 g/mol bis 300 g/mol, weiter bevorzugt mit einem Molgewicht im Bereich von 195 g/mol bis 290 g/mol, und am meisten bevorzugt im Bereich 200 bis 275 g/mol.

3. Zusammensetzung nach einem der vorangehenden Ansprüche, **dadurch gekennzeichnet, dass** Bestandteil (A) Cetylnonanoat und Stearylnonanoat enthält, wobei vorzugsweise das Massenverhältnis von Cetylnonanoat zu Stearylnonanoat im Bereich von 1 : 9 bis 9 : 1, bevorzugt im Bereich von 2 : 8 bis 8 : 2, weiter bevorzugt im Bereich von 3 : 7 bis 7 : 3, liegt.

4. Zusammensetzung nach einem Ansprüche 2 oder 3, **dadurch gekennzeichnet, dass** der, mehrere oder sämtliche Riechstoffe des Bestandteils (B) (i) ausgewählt sind aus der Gruppe bestehend aus:
(B) (i) n-Heptanol, Campher, alpha-Pinen, beta-Pinen, Limonen, 6-Methyl-5-hepten-2-on, Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Citral, Neral, Geranial, Benzylalkohol, p-Anisaldehyd, Menthol, Isoamylacetat, Isoamylbutyrat, cis-3-Hexenylacetat, Hexylacetat, Butylbutyrat, Citronellol, Nerol, Geraniol, 2-Phenylethylalkohol, Methylbenzoat, Agrunitril (3,7-Dimethyl-6-octen-1-nitril) und Vanillin.

5. Zusammensetzung nach einem Ansprüche 2 bis 4, **dadurch gekennzeichnet, dass** der, mehrere oder sämtliche Riechstoffe des Bestandteils (B) (ii) ausgewählt sind aus der Gruppe bestehend aus:
- Moschusriechstoffen,
alpha-n-Amylzimtaldehyd (MW = 202,30), alpha-iso-Amylzimtaldehyd (MW = 202,30), alpha-n-Hexylzimtaldehyd (MW = 216,32), alpha-iso-Hexylzimtaldehyd (MW = 216,32), Benzylsalicylat (MW = 228,25), cis-3-Hexenylsalicylat (MW = 220,27), Isoamylsalicylat (MW = 208,26), Hexylsalicylat (MW = 222,28), 2-Methyl-3-(4-tert-butylphenyl)propanal (MW = 204,31), 2-Methyl-3-(4-isopropylphenyl)propanal (MW = 190,28, Cyclamenaldehyd), 2,3,8,8-Tetramethyl-1,2,3,4,5,6,7,8-octahydro-2-naphthalenylmethylketon (MW = 234,38), 1-[1,2,3,4,6,7,8,8a-octahydro-1,2,8,8-tetramethylnaphtalen-2-yl]ethanon (MW = 234,38), Methyldihydrojasmonat (MW = 226,32), Linalylacetat (MW = 196.29), Ethyllinalylacetat (MW = 210,31), Nerolidol (MW = 222,37), Farnesol (MW = 222,37), Cedrylmethylether (MW = 236,40), Cedrylmethylketon (MW = 246,39), Cedrylacetat (MW = 264,41), (4aR,5R,7aS,9R)-Octahydro-2,2,5,8,8,9a-hexamethyl-4H-4a,9-Methanoazuleno(5,6-d) 1,3-dioxol) (MW = 278,44), Hexahydro-1',1',5',5'-tetramethyl-spiro[1,3-dioxolan,2,8' (5'H)-[2H-2,4a]-methanonaphthalin (MW = 264,41 Ethylendioxi-3H-isolongifolan), 2-Methyl-4-(2,2,3-trimethyl-3-cyclopenten-1-yl)butanol (MW = 196,34), 5-(2,2,3-Trimethyl-3-cyclopenten-1-yl)-3-methylpentan-2-ol (MW = 210,36), 2-Ethyl-4-(2,2,3-trimethyl-3-cyclopenten-1-yl)-2-buten-1-ol (MW = 208,35), 3-Methyl-5-(2,2,3-trimethyl-3-cyclopenten-1-yl)-4-penten-2-ol (MW = 208,35), 3,3-Dimethyl-5-(2,2,3-trimethyl-3-cyclopenten-1-yl)-4-penten-2-ol (MW = 222,37), 3-Isocamphylcyclohexanol (MW = 236,40),1-(2,2,6-Trimethylcyclohexyl)hexan-3-ol (MW = 226,41), Cyclododecylmethylether (MW = 198,35), (Ethoxymethoxy)cyclododecan (MW = 242,41), 1-Methyl-4-(4-methyl-3-penten-1-yl)-3-cyclohexencarboxaldehyd (MW = 206,33), 4-(4-Hydroxy-4-methylpentyl)-3-cyclohexencarboxaldehyd (MW = 210,32), 2-Methyl-4-(2,2,6-trimethyl-1-cyclohexen-1-yl)-2-butenal (MW = 206,33), Decahydro-beta-naphthylacetat (MW = 196,29), Allyl-3-cyclohexylpropionat (MW = 196,29), Allylcyclohexyloxyacetat (MW = 198,26), Citraldiethylacetal (MW = 226,36), Benzylbenzoat (MW = 212,25), Benzylcinnamat (MW = 238,29), 3a,6,6,9a-Tetramethyldodecahydronaphtho[2,1-b]furan (MW = 236,40), alpha-Iron (MW = 206,33), beta-Iron (MW = 206,33), alpha-n-Methylionon (MW = 206,33), beta-n-Methylionon (MW = 206,33), alpha-Isomethylionon (MW = 206,33), beta-Isomethylionon (MW = 206,33) und Allylionon (MW 232,35). 2,3,8,8-Tetramethyl-1,2,3,4,5,6,7,8-octahydro-2-naphthalenylmethylketon (MW = 234,38), 1-[1,2,3,4,6,7,8,8a-octahydro-1,2,8,8-tetramethylnaphtalen-2-yl]ethanon (MW = 234,38), Isobornylacetat (MW = 196,29), alpha-Ionon (MW = 192,30), beta-Ionon (MW = 192,30), gamma-Ionon (MW = 192,30), alpha-Damascon (MW = 192,30), beta-Damascon (MW = 192,30), delta-Damascon (MW = 192,30), 1-(2,4,4-Trimethyl-2-cyclohexen-1-yl)-2-buten-1-on (MW = 192,30), Cedrol (MW = 222,40), gamma-Dodecalacton (MW = 198,30), 2-Methyl-3-(3,4-methylendioxyphenyl)propanal (MW = 192,22) und Methyldihydrojasmonat (MW = 226,31).

6. Zusammensetzung nach einem der vorangehenden Ansprüche, als zusätzlichen Bestandteil enthaltend eine wirksame Menge
(C) eines oder mehrerer kosmetisch akzeptabler Lösungsvermittler für Bestandteil (B), vorzugsweise für Bestandteil (B*), (B) (i) und/oder (B) (ii), vorzugsweise gewählt aus der Gruppe bestehend ausEthanol, Dipropylenglycol, Diethylphtalat, Triethylcitrat, Isopropylmyristat und Triacetin, dabei bevorzugt ist Dipropylenglycol.

7. Zusammensetzung nach einem der vorangehenden Ansprüche, als zusätzlichen Bestandteil enthaltend
(D) ein oder mehrere Di- oder Triole mit 3 bis 12 C-Atomen, vorzugsweise gewählt aus der Gruppe bestehend aus Glycerin, 1,2-Propylenglykol, 1,2-Butylenglykol, 1,3-Butylenglykol und Alkandiolen mit 5 bis 12 C-Atomen.

8. Kosmetisches Produkt enthaltend eine Zusammensetzung wie in einem der vorangehenden Ansprüche definiert.

9. Zusammensetzung nach einem der Ansprüche 1-7 oder kosmetisches, vorzugsweise topisches, Produkt, nach Anspruch 8
umfassend
(A) Cetylnonanoat und/oder Stearylnonanoat,
und
(B) einen oder mehrere Riechstoffe,
enthaltend
(A) Cetylnonanoat und/oder Stearylnonanoat in einer Gesamtmenge von 0,25 - 30 Gew.-%, bevorzugt 0,25 - 20 Gew.-%, weiter bevorzugt 0,5-15 Gew.-%, besonders bevorzugt 0,5-10 Gew.-%, und ganz besonders bevorzugt 1-10 Gew.-%, und/oder
(B) einen oder mehrere Riechstoffe in einer Menge von 0,15-5 Gew.-%, bevorzugt 0,2 - 3 Gew.-%, weiter bevorzugt 0,3 - 3 Gew.-%, besonders bevorzugt 0,3 - 2,5 Gew.-%, und ganz besonders bevorzugt 0,3 - 2 Gew.-%, wobei vorzugsweise Bestandteil (B)
- (B) (i) einen, 2, 3, 4, 5 oder mehr Riechstoffe mit einem Molgewicht im Bereich von 100 g/mol bis 175 g/mol (Topnote) enthält, vorzugsweise mit einem Molgewicht im Bereich von 110 g/mol bis 160 g/mol, bevorzugt im Bereich von 115 g/mol bis 160 g/mol, besonders bevorzugt im Bereich von 120 g/mol bis 155 g/mol, und/oder
- (B) (ii) einen, 2, 3, 4, 5 oder mehr Riechstoffe mit einem Molgewicht von größer oder gleich 190 g/mol (Fondnote) enthält, bevorzugt im Bereich von 190 g/mol bis 300 g/mol, weiter bevorzugt mit einem Molgewicht im Bereich von 195 g/mol bis 290 g/mol, und am meisten bevorzugt im Bereich 200 bis 275 g/mol.
und/oder
(C) (ii)Dipropylenglycol, Diethylphtalat, Triethylcitrat, Isopropylmyristat und Triacetin, wobei die Gesamtmenge des Bestandteils (C) (ii) bis zu 80 Gew.-%, bevorzugt von 0,5 bis 60 Gew.-%, bevorzugt von 1 bis 50 Gew.-%, weiter bevorzugt von 5 bis 40 Gew.-%, jeweils bezogen auf das Gesamtgewicht des Bestandteils (B) beträgt,
und/oder
(D) ein oder mehrere Di- oder Triole mit 3 bis 12 C-Atomen in einer Gesamtmenge von 0,2 bis 20 Gew.-%, bevorzugt von 0,5 bis 10 Gew.-%, weiter bevorzugt von 1 bis 5 Gew.-%,
jeweils bezogen auf das Gesamtgewicht der Zusammensetzung bzw. des Produktes.

10. Verfahren zur
- Reduzierung der Verdampfung von Riechstoffen in einer Riechstoffzusammensetzung, insbesondere unmittelbar nach der Applikation,
- zeitlichen Stabilisierung des Geruchsprofils einer Riechstoffzusammensetzung,
- Erhöhung oder zeitlichen Stabilisierung des geruchlichen Impacts einer Riechstoffzusammensetzung,
- Verlängerung der Haftung von Riechstoffen einer Riechstoffzusammensetzung, insbesondere der Kopfnote und/oder Fondnote, auf Haut und/oder Haar, insbesondere auf menschlicher Haut,
und/oder
- Vermittlung oder Verstärkung eines angenehmen Hautgefühls einer Riechstoffzusammensetzung,
mit folgendem Schritt:
Mischen von
(A) einer Bestandteil (B) fixierenden Menge an Cetylnonanoat und/oder Stearylnonanoat und
(B) einem oder mehreren Riechstoffen,
wobei vorzugsweise das Massenverhältnis des Bestandteils (A) zur Gesamtmasse des Bestandteils (B) im Bereich von 1 : 20 bis 200 : 1 liegt, bevorzugt im Bereich von 1 : 12 bis 100 : 1, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1.

11. Verfahren zur
- Vermittlung, Verstärkung oder Modifizierung eines Geruchs auf der Haut,
- Verlängerung der Haftung von Riechstoffen einer Riechstoffzusammensetzung, insbesondere der Kopfnote und/oder Fondnote, auf Haut und/oder Haar, insbesondere auf menschlicher Haut,
und/oder
- Vermittlung oder Verstärkung eines angenehmen Hautgefühls einer Riechstoffizusammensetzung,
mit folgendem Schritt:
- Applizieren einer Zusammensetzung nach einem der Ansprüche 1 bis 7 oder eines Produktes nach Anspruch 8 oder 9 auf Haut und/oder Haar.

12. Verwendung von Cetylnonanoat, Stearylnonanoat oder Mischungen von Cetylnonanoat und Stearylnonanoat als Fixateur für Riechstoffe und Parfümöle.

13. Verwendung von Cetylnonanoat, Stearylnonanoat oder Mischungen von Cetylnonanoat und Stearylnonanoat als Mittel zur
- Reduzierung der Verdampfung von Riechstoffen in einer Riechstoffzusammensetzung, insbesondere unmittelbar nach der Applikation;
- zeitlichen Stabilisierung des Geruchsprofils einer Riechstoffzusammensetzung;
- Erhöhung oder zeitlichen Stabilisierung des geruchlichen Impacts einer Riechstoffzusammensetzung;
- Verlängerung der Haftung von Riechstoffen einer Riechstoffzusammensetzung, insbesondere der Kopfnote und/oder Fondnote, auf Haut und/oder Haar, insbesondere auf menschlicher Haut;
und/oder
- Vermittlung oder Verstärkung eines angenehmen Hautgefühls einer Riechstoffzusammensetzung.

## Claims

1. Composition comprising or consisting of
(A) Cetyl nonanoate and/or stearyl nonanoate,
and
(B) one or several fragrance(s), preferably a fragrance mixture comprising 2, 3, 4, 5, 6, 7, 8, 9, 10 or more fragrances,
wherein component (A) is contained in an amount that fixes the fragrance or several or all of the fragrances of component (B),
wherein preferably the mass ratio of component (A) to the total mass of component (B) is in the range of 1 : 20 to 200 : 1, preferably in the range of 1 : 12 to 100 : 1, further preferably in the range of 1 : 6 to 50 : 1, particularly preferably in the range of 1 : 5 to 33 : 1, and particularly preferably in the range of 1 : 2 to 25 : 1, subject to the proviso that the composition is not a gland secretion of *Trichio-campus grandis* consisting of 7% hexadecyl pentanoate, 2% octyl octanoate, 1% hexadecyl nonanoate, 1% octyl decanoate, 3% hexyl hexadecanoate, 24% 2-hexenyl hexadecanoate, 14% 2-octenyl hexadecanoate, 4% methyl icosenoate, 3% methyl docosanoate, 13% methyl docosenoate, 2% methyl tetracosanoate, 2% methyl tetracosenoate, 1% hexadecanal, 2% tetracosanal, 11% of an acetogenin, 5% impurity, as well as traces of pentacosane, heptacosane, nonacosane, methyl hexadecanoate, isopropyl hexadecanoate, hexenyl hexadecanoate, heptenyl hexadecanoate, methyl octadecanoate, methyl octadecenoate, isopropyl octadecanoate, (Z)-3-octenal and octadecanal.

2. Composition according to claim 1, **characterised in that** component (B)
(B) (i) comprises one, 2, 3, 4, 5 or more fragrances with a molecular weight in the range of 100 g/mol to 175 g/mol (top note), preferably with a molecular weight in the range of 110 g/mol to 160 g/mol, more preferably in the range of 115 g/mol to 160 g/mol, particularly preferably in the range of 120 g/mol to 155 g/mol, and/or
(B) (ii) comprises one, 2, 3, 4, 5 or more fragrances with a molecular weight of greater than or equal to 190 g/mol (base note), preferably with a molecular weight in the range of 190 g/mol to 300 g/mol, further preferably with a molecular weight in the range of 195 g/mol to 290 g/mol, and most preferably in the range of 200 to 275 g/mol.

3. Composition according to any one of the preceding claims, **characterised in that** component (A) comprises cetyl nonanoate and stearyl nonanoate, wherein preferably the mass ratio of cetyl nonanoate to stearyl nonanoate is in the range of 1 : 9 to 9 : 1, preferably in the range of 2 : 8 to 8 : 2, further preferably in the range of 3 : 7 to 7 : 3.

4. Composition according to claim 2 or 3, **characterised in that** the fragrance or several or all of the fragrances of component (B) (i) is/are selected from the group consisting of:
(B) (i)n-heptanol, camphor, alpha-pinene, beta-pinene, limonene, 6-methyl-5-hepten-2-one, octanal, eucalyptol (1,8-cineole), rose oxide, 3-hexenol, dihydromyrcenol (2,6-dimethyl-7-octen-2-ol), menthone, isomenthone, 2,6-dimethyl-5-hepten-1-al (melonal), 3-hexenyl methyl carbonate, benzaldehyde, linalool, tetrahydrolinalool, citral, neral, geranial, benzyl alcohol, p-anisaldehyde, menthol, isoamyl acetate, isoamyl butyrate, cis-3-hexenyl acetate, hexyl acetate, butyl butyrate, citronellol, nerol, geraniol, 2-phenylethyl alcohol, methyl benzoate, agrunitril (3,7-dimethyl-6-octene-1-nitril) and vanillin.

5. Composition according to any one of the claims 2 to 4, **characterised in that** the fragrance or several or all of the fragrances of component (B) (ii) is/are selected from the group consisting of:
- musk fragrances,
alpha-n-amylcinnamaldehyde (MW = 202,30), alpha-iso-amylcinnamaldehyde (MW = 202.30), alpha-n-hexylcinnamaldehyde (MW = 216.32), alpha-iso-hexylcinnamaldehyde (MW = 216.32), benzyl salicylate (MW = 228.25), cis-3-hexenyl salicylate (MW = 220.27), isoamyl salicylate (MW = 208.26), hexyl salicylate (MW = 222.28), 2-methyl-3-(4-tert-butylphenyl)propanal (MW = 204.31), 2-methyl-3-(4-isopropylphenyl)propanal (MW = 190.28, cyclamen aldehyde), 2,3,8,8-tetramethyl-1,2,3,4,5,6,7,8-octahydro-2-naphthalenyl methyl ketone (MW = 234.38), 1-[1,2,3,4,6,7,8,8a-octahydro-1,2,8,8-tetramethyl naphtalen-2-yl]ethanone (MW = 234.38), methyl dihydrojasmonate (MW = 226.32), linalyl acetate (MW = 196.29), ethyl linalyl acetate (MW = 210.31), nerolidol (MW = 222.37), farnesol (MW = 222.37), cedryl methyl ether (MW = 236.40), cedryl methyl ketone (MW = 246.39), cedryl acetate (MW = 264.41), (4aR,5R,7aS,9R)-octahydro-2,2,5,8,8,9a-hexamethyl-4H-4a,9-methanoazuleno(5,6-d) 1,3-dioxole (MW = 278.44), hexahydro-1',1',5',5'-tetramethyl-spiro(1,3-dioxolane-2,8'-(5'H)-[2H-2,4a]-methanonaphthalene (MW = 264.41 ethylenedioxy-3H-isolongifolene), 2-methyl-4-(2,2,3-trimethyl-3-cyclopenten-1-yl)butanol (MW = 196.34), 5-(2,2,3-trimethyl-3-cyclopenten-1-yl)-3-methylpentan-2-ol (MW = 210.36), 2-ethyl-4-(2,2,3-trimethyl-3-cyclopenten-1-yl)-2-buten-1-ol (MW = 208.35), 3-methyl-5-(2,2,3-trimethyl-3-cyclopenten-1-yl)-4-penten-2-ol (MW = 208.35), 3,3-dimethyl-5-(2,2,3-trimethyl-3-cyclopenten-1-yl)-4-penten-2-ol (MW = 222.37), 3-isocamphyl cyclohexanol (MW = 236.40), 1-(2,2,6-trimethylcyclohexyl)hexan-3-ol (MW = 226.41), cyclododecyl methyl ether (MW = 198.35), (ethoxymeth-oxy)cyclododecane (MW = 242.41), 1-methyl-4-(4-methyl-3-penten-1-yl)-3-cyclohexene carboxaldehyde (MW = 206.33), 4-(4-hydroxy-4-methylpentyl)-3-cyclohexene carboxaldehyde (MW = 210.32), 2-methyl-4-(2,2,6-trimethyl-1-cyclohexen-1-yl)-2-butenal (MW = 206.33), decahydro-beta-naphthyl acetate (MW = 196.29), allyl-3-cyclohexyl propionate (MW = 196.29), allyl cyclohexyl oxyacetate (MW = 198.26), citral diethyl acetal (MW = 226.36), benzyl benzoate (MW = 212.25), benzyl cinnamate (MW = 238.29), 3a,6,6,9a-tetramethyl dodecahydronaphtho[2,1-b]furan (MW = 236.40), alpha-iron (MW = 206.33), beta-iron (MW = 206.33), alpha-n-methyl ionone (MW = 206.33), beta-n-methyl ionone (MW = 206.33), alpha-isomethyl ionone (MW = 206.33), beta-isomethyl ionone (MW = 206.33) and allyl ionone (MW 232.35). 2,3,8,8-Tetramethyl-1,2,3,4,5,6,7,8-octahydro-2-naphthalenyl methyl ketone (MW = 234.38), 1-[1,2,3,4,6,7,8,8a-octahydro-1,2,8,8-tetramethylnaphtalen-2-yl]ethanone (MW = 234.38), isobornyl acetate (MW = 196.29), alpha-ionone (MW = 192.30), beta-ionone (MW = 192.30), gamma-ionone (MW = 192.30), alpha-damascone (MW = 192.30), beta-damascone (MW = 192.30), delta-damascone (MW = 192.30), 1-(2,4,4-trimethyl-2-cyclohexen-1-yl)-2-buten-1-one (MW = 192.30), cedrol (MW = 222.40), gamma-dodecalactone (MW = 198.30), 2-methyl-3-(3,4-methylenedioxyphenyl)propanal (MW = 192.22) and methyl dihydrojasmonate (MW = 226.31).

6. Composition according to any one of the preceding claims, comprising as an additional component an effective amount of
(C) one or several cosmetically acceptable solubilising agent(s) for component (B), preferably for component (B*), (B) (i) and/or (B) (ii), preferably selected from the group consisting of ethanol, dipropylene glycol, diethyl phtalate, triethyl citrate, isopropyl myristate and triacetin, thereby preferred is dipropylene glycol.

7. Composition according to any one of the preceding claims, comprising as an additional component
(D) one or several di- or triols with 3 to 12 C-atoms, preferably selected from the group consisting of glycerol, 1,2-propylene glycol, 1,2-butylene glycol, 1,3-butylene glycol and alkane diols with 5 to 12 C-atoms.

8. Cosmetic product comprising a composition as defined in any one of the preceding claims.

9. Composition according to any one of the claims 1 - 7 or cosmetic, preferably topical, product, according to claim 8
comprising
(A) cetyl nonanoate and/or stearyl nonanoate, and
(B) one or several fragrance(s), comprising
(A) cetyl nonanoate and/or stearyl nonanoate in a total amount of 0.25 - 30 wt.-%, preferably 0.25 - 20 wt.-%, further preferably 0.5-15 wt.-%, particularly preferably 0.5 - 10 wt.-%, and particularly preferably 1-10 wt.-% and/or
(B) one or several fragrance(s) in an amount of 0.15 - 5 wt.-%, preferably 0.2 - 3 wt.-%, further preferably 0.3 - 3 wt.-%, particularly preferably 0.3-2.5 wt.-%, and particularly preferably 0.3 - 2 wt.%, wherein preferably component (B)
- (B) (i)comprises one, 2, 3, 4, 5 or more fragrance(s) with a molecular weight in the range of 100 g/mol to 175 g/mol (top note), preferably with a molecular weight in the range of 110 g/mol to 160 g/mol, preferably in the range of 115 g/mol to 160 g/mol, particularly preferably in the range of 120 g/mol bis 155 g/mol,
and/or
- (B) (ii) comprises one, 2, 3, 4, 5 or more fragrance(s) with a molecular weight of greater than or equal to 190 g/mol (base note), preferably in the range of 190 g/mol to 300 g/mol, further preferably with a molecular weight in the range of 195 g/mol to 290 g/mol, and most preferably in the range of 200 to 275 g/mol.
and/or
(C) (ii) dipropylene glycol, diethyl phtalate, triethyl citrate, isopropyl myristate and triacetin, wherein the total amount of component (C) (ii) is up to 80 wt.-%, preferably from 0.5 to 60 wt.-%, preferably from 1 to 50 wt.-%, further preferably from 5 to 40 wt.-%, with respect to the total weight of component (B), respectively, and/or
(D) one or several di- or triols with 3 to 12 C-atoms in a total amount of 0.2 to 20 wt.-%, preferably from 0.5 to 10 wt.-%, further preferably from 1 to 5 wt.-%,
with respect to the total weight of the composition and the product, respectively.

10. Method for
- the reduction of the evaporation of fragrances in a fragrance composition, particularly immediately after application,
- temporal stabilisation of the olfactory profile of a fragrance composition,
- increase or temporal stabilisation of the olfactory impact of a fragrance composition,
- prolongation of the adhesion of fragrances of a fragrance composition, particularly of the top note and/or base note, to skin and/or hair, particularly to human skin, and/or
- conveyance or enhancement of a pleasant feeling on the skin of a fragrance composition,
with the following step:
Mixing of
(A) an amount of cetyl nonanoate and/or stearyl nonanoate that fixes component (B) and
(B) one or several fragrance(s),
wherein preferably the mass ratio of component (A) to the total mass of component (B) is in the range of 1 : 20 to 200 : 1, preferably in the range of 1 : 12 to 100 : 1, further preferably in the range of 1 : 6 to 50 : 1, particularly preferably in the range of 1 : 5 to 33 : 1, and particularly preferably in the range of 1 : 2 to 25:1.

11. Method for
- conveyance, enhancement or modification of a scent on the skin,
- prolongation of the adhesion of fragrances of a fragrance composition, particularly of the top note and/or base note, to skin and/or hair, particularly to human skin,
and/or
- conveyance or enhancement of a pleasant feeling on the skin of a fragrance composition,
with the following step:
- application of a composition according to any one of the claims 1 to 7 or of a product according to claim 8 or 9 to the skin and/or hair.

12. Use of cetyl nonanoate, stearyl nonanoate or mixtures of cetyl nonanoate and stearyl nonanoate as fixative for fragrances and perfume oils.

13. Use of cetyl nonanoate, stearyl nonanoate or mixtures of cetyl nonanoate and stearyl nonanoate as a means of
- reduction of the evaporation of fragrances in a fragrance composition, particularly immediately after application;
- temporal stabilisation of the olfactory profile of a fragrance composition;
- increase or temporal stabilisation of the olfactory impact of a fragrance composition;
- prolongation of the adhesion of fragrances of a fragrance composition, particularly of the top note and/or base note, to skin and/or hair, particularly to human skin;
and/or
- conveyance or enhancement of a pleasant feeling on the skin of a fragrance composition.

## Revendications

1. Composition comprenant ou consistant en
(A) nonanoate de cétyle et/ou nonanoate de stéaryle,
et
(B) une ou plusieurs substances odoriférantes, de préférence un mélange de substances odoriférantes comprenant 2, 3, 4, 5, 6, 7, 8, 9, 10 substances odoriférantes ou plus,
dans laquelle le composant (A) est contenu en une quantité fixatrice pour la, plusieurs ou toutes les substances odoriférantes du composant (B),
dans lequel, de préférence, le rapport de masse du composant (A) à la masse totale du composant (B) se situe dans la plage allant de 1 : 20 à 200 : 1, de préférence dans la plage allant de 1 : 12 à 100 : 1, encore de préférence dans la plage allant de 1 : 6 à 50 : 1, de manière particulièrement préférée dans la plage allant de 1 : 5 à 33 : 1, et de manière tout particulièrement préférée dans la plage allant de 1 : 2 à 25 : 1,
sous réserve que la composition n'est pas de sécrétion glandulaire de *Trichio-campus grandis,* se composant de 7 % de pentanoate d'hexadécyle, de 2% d'octanoate d'octyle, de 1 % de nonanoate d'hexadécyle, de 1 % de décanoate d'octyle, de 3 % d'hexadécanoate d'hexyle, de 24 % d'hexadécanoate de 2-hexényle, de 14 % d'hexadécanoate de 2-octényle, de 4 % d'icosénoate de méthyle, de 3 % de docosanoate de méthyle, de 13 % de docosénoate de méthyle, de 2 % de tétracosanoate de méthyle, de 2 % de tétracosénoate de méthyle, de 1 % d'hexadécanal, de 2 % de tétracosanal, de 11 % d'une acétogénine, de 5 % d'impureté, ainsi que de traces de pentacosane, d'heptacosane, de nonacosane, d'hexadécanoate de méthyle, d'hexadécanoate d'isopropyle, d'hexadé-canoate d'hexényle, d'hexadécanoate d'heptényle, d'octadécanoate de méthyle, d'octadécénoate de méthyle, d'octadécanoate d'isopropyle, de (Z)-3-octénal et d'octadécanal.

2. Composition selon la revendication 1, **caractérisée par le fait que** le composant (B)
(B) (i) contient une, 2, 3, 4, 5 substances odoriférantes ou plus, ayant un poids moléculaire compris entre 100 g/mole et 175 g/mole (note dominante), de préférence un poids moléculaire compris entre 110 g/mole et 160 g/mole, de préférence compris entre 115 g/mole et 160 g/mole, de manière particulièrement préférée compris entre 120 g/mole et 155 g/mole,
et/ou
(B) (ii) contient une, 2, 3, 4, 5 substances odoriférantes ou plus, ayant un poids moléculaire supérieur ou égal à 190 g/mole (note de base), de préférence un poids moléculaire compris entre 190 g/mole et 300 g/mole, encore de préférence un poids moléculaire compris entre 195 g/mole et 290 g/mole, et, de la manière la plus préférée, compris entre 200 et 275 g/mole.

3. Composition selon l'une quelconque des revendications précédente, **caractérisée par le fait que** le composant (A) contient du nonanoate de cétyle et du nonanoate de stéaryle, de préférence le rapport de masse du nonanoate de cétyle au nonanoate de stéaryle se situant dans la plage allant de 1 : 9 à 9 : 1, de préférence dans la plage allant de 2 : 8 à 8 : 2 , encore de préférence dans la plage allant de 3 : 7 à 7 : 3.

4. Composition selon l'une quelconque des revendications 2 ou 3, **caractérisée par le fait que** la, plusieurs ou toutes les substances odoriférantes du composant (B) (i) sont choisies dans le groupe constitué par:
(B) (i) le n-heptanol, le camphre, l'alpha-pinène, le bêta-pinène, le limonène, le 6-méthyl-5-heptène-2-one, l'octanal, l'eucalyptol (1,8-cinéole), l'oxyde de rose, le 3-hexénol, le dihydromyrcénol (2,6-diméthyl-7-octène-2-ol), la menthone, l'isomenthone, le 2,6-diméthyl-5-heptène-1-al (mélonal), le carbonate de 3-hexénylméthyle, le benzaldéhyde, le linalol, le tétrahydro-linalol, le citral, le néral, le géranial, l'alcool benzylique, l'aldéhyde p-anisique, le menthol, l'acétate d'isoamyle, le butyrate d'isoamyle, l'acétate de cis-3-hexényle, l'acétate d'hexyle, le butyrate de butyle, le citronellol, le nérol, le géraniol, l'alcool 2-phényléthylique, le benzoate de méthyle, l'agrunitrile (3,7-diméthyl-6-octène-1-nitrile) et la vanilline.

5. Composition selon l'une quelconque des revendications 2 à 4, **caractérisée par le fait que** la, plusieurs ou toutes les substances odoriférantes du composant (B) (ii) sont choisies dans le groupe constitué par:
- les substances odoriférantes de musc,
l'alpha-n-amyl-cinnamaldéhyde (PM = 202,30), l'alpha-iso-amyl-cinnamaldéhyde (PM = 202,30), l'alpha-n-hexyl-cinnamaldéhyde (PM = 216,32), l'alpha-iso-hexyl- cinnamaldéhyde (PM = 216,32), le salicylate de benzyle (PM = 228,25), le salicylate de cis-3-hexényle (PM = 220,27), le salicylate d'isoamyle (PM = 208,26), le salicylate d'hexyle (PM = 222,28), le 2-méthyl-3-(4-tert-butylphényl)propanal (PM = 204,31), le 2-méthyl-3-(4-isopropylphényl)propanal (PM = 190,28, aldéhyde de cyclamen), la 2,3,8,8-tétraméthyl-1,2,3,4,5,6,7,8-octahydro-2-naphtalényle méthylcétone (PM = 234,38), la 1-[1,2,3,4,6,7,8,8a-octahydro-1,2,8,8-tétraméthylnaphtalène-2-yl]éthanone (PM = 234,38), le dihydrojasmonate de méthyle (PM = 226,32), l'acétate de linalyle (PM = 196,29), l'acétate d'éthyl linalyle (PM = 210,31), le nérolidol (PM = 222,37), le farnésol (PM = 222,37), l'éther méthylique de cédryle (PM = 236,40), la cédryl méthyl cétone (PM = 246,39), l'acétate de cédryle (PM = 264,41), le (4aR,5R,7aS,9R)-octahydro-2,2,5,8,8,9a-hexaméthyl-4H-4a,9-méthanoazuléno(5,6-d) 1,3-dioxole) (PM = 278,44), l'hexahydro-1',1',5',5'-tétraméthyl-spiro[1,3-dioxolane,2,8'(5'H)-[2H-2,4a]-methanonaphtalène (PM = 264,41 éthylène dioxy-3H-isolongifolane), le 2-méthyl-4-(2,2,3-triméthyl-3-cyclopentène-1-yl)butanol (PM = 196,34), le 5-(2,2,3-triméthyl-3-cyclopentène-1-yl)-3-méthylpentan-2-ol (PM = 210,36), le 2-éthyl-4-(2,2,3-triméthyl-3-cyclopentène-1-yl)-2-butène-1-ol (PM = 208,35), le 3-méthyl-5-(2,2,3-triméthyl-3-cyclopentène-1-yl)-4-pentène-2-ol (PM = 208,35), le 3,3-diméthyl-5-(2,2,3-triméthyl-3-cyclopentène-1-yl)-4-pentène-2-ol (PM = 222,37), le 3-isocamphylcyclohexanol (PM = 236,40), le 1-(2,2,6-triméthylcyclohexyl)hexane-3-ol (PM = 226,41), le cyclododécylméthylether (PM = 198,35) l'(éthoxyméthoxy)cyclododécane (PM = 242,41), le 1-méthyl-4-(4-méthyl-3-pentène-1-yl)-3-cyclohexène carboxaldehyde (PM = 206,33), le 4-(4-hydroxy-4-méthylpentyle)-3-cyclohexène carboxaldehyde (PM = 210,32), le 2-méthyl-4-(2,2,6-triméthyl-1-cyclohexène-1-yl)-2-buténal (PM = 206,33), le décahydro-bêta-naphtyl acétate (PM = 196,29), l'allyl-3-cyclohexyl propionate (PM = 196,29), l'allyl cyclohexyl oxyacétate (PM = 198,26) le citral-diéthylacétal (PM = 226,36), le benzoate de benzyle (PM = 212,25), le cinnamate de benzyle (PM = 238,29), le 3a,6,6,9a-tétraméthyl dodécahydronaphto[2,1-b]furane (PM = 236,40), l'alpha-irone (PM = 206,33), la bêta-irone (PM = 206,33), l'alpha-n-méthylionone (PM = 206,33), la bêta-n-méthylionone (PM = 206,33), l'alpha-isométhylionone (PM = 206,33), la bêta-isométhylionone (PM = 206,33) et l'allylionone (PM 232,35), la 2,3,8,8-tétraméthyl-1,2,3,4,5,6,7,8-octahydro-2-naphtalénylméthylcétone (PM = 234,38), la 1-[1,2,3,4,6,7,8,8a-octahydro-1,2,8,8-tetraméthylnaphtalène-2-yl]éthanone (PM = 234,38), l'acétate d'isobor-nyle (PM = 196,29), l'alpha-ionone (PM = 192,30), la bêta-ionone (PM = 192,30), la gamma-ionone (PM = 192,30), l'alpha-damascone (PM = 192,30), la bêta-damascone (PM = 192,30), la delta-damascone (PM = 192,30), le 1-(2,4,4-triméthyl-2-cyclohexène-1-yl)-2-butène-1-one (PM = 192,30), le cédrol (PM = 222,40), la gamma-dodécalactone (PM = 198,30), le 2-méthyl-3-(3,4-méthylène dioxyphényl)propanal (PM = 192,22) et le dihydrojasmonate de méthyle (PM = 226,31).

6. Composition selon l'une quelconque des revendications précédentes, comprenant, en tant que composant supplémentaire, une quantité efficace (C) d'un ou de plusieurs agent(s) de solubilisation cosmétiquement acceptable(s) pour le composant (B), de préférence pour le composant (B*), (B) (i) et/ou (B) (ii)), de préférence choisi(s) dans le groupe constitué par l'éthanol, le dipropylèneglycol, le phtalate de diéthyle, le citrate de triéthyle, le myristate d'isopropyle et la triacétine, le dipropylèneglycol y étant préféré.

7. Composition selon l'une quelconque des revendications précédentes, comprenant en tant que composant supplémentaire
(D) un ou plusieurs di- ou triols ayant 3 à 12 atomes de carbone, de préférence choisis dans le groupe constitué par le glycérol, le 1,2-propylène glycol, le 1,2-butylène glycol, le 1,3-butylène glycol et les alcanediols ayant 5 à 12 atomes de carbone.

8. Produit cosmétique contenant une composition telle que définie dans l'une quelconque des revendications précédentes.

9. Composition selon l'une quelconque des revendications 1 à 7 ou produit cosmétique, de préférence topique, selon la revendication 8,
comprenant
(A) du nonanoate de cétyle et/ou du nonanoate de stéaryle, et
(B) une ou plusieurs substances odoriférantes,
contenant
(A) du nonanoate de cétyle et/ou du nonanoate de stéaryle en une quantité totale comprise entre 0,25 et 30 % en poids, de préférence entre 0,25 et 20 % en poids, encore de préférence entre 0,5 et 15 % en poids, de manière particulièrement préférée entre 0,5 et 10 % en poids et de manière tout particulièrement préférée entre 1 et 10 % en poids, et/ou
(B) une ou plusieurs substances odoriférantes en une quantité comprise entre 0,15 à 5 % en poids, de préférence entre 0,2 et 3 % en poids, encore de préférence entre 0,3 et 3 % en poids, de manière particulièrement préférée entre 0,3 et 2,5 % en poids et de manière tout particulièrement préférée entre 0,3 et 2 % en poids, de préférence le composant (B)
- (B) (i) contient une, 2, 3, 4, 5 substances odoriférantes ou plus, ayant un poids moléculaire compris entre 100 g/mole et 175 g/mole (note dominante), de préférence un poids moléculaire compris entre 110 g/mole et 160 g/mole, de préférence compris entre 115 g/mole et 160 g/mole, de manière particulièrement préférée compris entre 120 g/mole et 155 g/mole,
et/ou
- (B) (ii) contient une, 2, 3, 4, 5 substances odoriférantes ou plus, ayant un poids moléculaire supérieur ou égal à 190 g/mole (note de base), de préférence compris entre 190 g/mole et 300 g/mole, encore de préférence un poids moléculaire compris entre 195 g/mole et 290 g/mole, et, de la manière la plus préférée, compris entre 200 et 275 g/mole,
et/ou
(C) (ii) le dipropylèneglycol, le phtalate de diéthyle, le citrate de triéthyle, le myristate d'isopropyle et la triacétine, la quantité totale du composant (C) (ii) allant jusqu'à 80 % en poids, de préférence est comprise entre 0,5 et 60 % en poids, de préférence entre 1 et 50 % en poids, encore de préférence entre 5 et 40 % en poids, respectivement par rapport au poids total du composant (B), et/ou
(D) un ou plusieurs di- ou triols ayant 3 à 12 atomes de carbone, en une quantité totale comprise entre 0,2 et 20 % en poids, de préférence entre 0,5 et 10 % en poids, encore de préférence entre 1 et 5 % en poids, respectivement par rapport au poids total de la composition ou bien du produit.

10. Procédé
- de réduction de l'évaporation de substances odoriférantes dans une composition de substances odoriférantes, en particulier immédiatement après l'application,
- de stabilisation dans le temps du profil olfactif d'une composition de substances odoriférantes,
- d'augmentation ou de stabilisation dans le temps de l'impact olfactif d'une composition de substances odoriférantes,
- de prolongement de l'adhésion de substances odoriférantes d'une composition de substances odoriférantes, en particulier de la note de tête et/ou note de base, à la peau et/ou aux cheveux, en particulier à la peau humaine,
et/ou
- pour conférer ou renforcer une sensation agréable sur la peau d'une
composition de substances odoriférantes,
comprenant l'étape suivante:
mélanger
(A) une quantité de nonanoate de cétyle et/ou de nonanoate de stéaryle,
fixant le composant (B)
et
(B) une ou plusieurs substances odoriférantes,
dans lequel, de préférence, le rapport de masse du composant (A) à la masse totale du composant (B) se situe dans la plage allant de 1 : 20 à 200 : 1, de préférence dans la plage allant de 1 : 12 à 100 : 1, encore de préférence dans la plage allant de 1 : 6 à 50 : 1, de manière particulièrement préférée dans la plage allant de 1 : 5 à 33 : 1, et de manière tout particulièrement préférée dans la plage allant de 1 : 2 à 25 : 1.

11. Procédé
- pour conférer, renforcer ou modifier une odeur sur la peau,
- de prolongement de l'adhésion de substances odoriférantes d'une composition de substances odoriférantes, en particulier de la note de tête et/ou note de base, à la peau et/ou aux cheveux, en particulier à la peau humaine,
et/ou
- pour conférer ou renforcer une sensation agréable sur la peau d'une
composition de substances odoriférantes,
comprenant l'étape suivante
- appliquer une composition selon l'une quelconque des revendications 1 à 7 ou un produit selon la revendication 8 ou 9 sur la peau et/ou les cheveux.

12. Utilisation de nonanoate de cétyle, de nonanoate de stéaryle ou de mélanges de nonanoate de cétyle et de nonanoate de stéaryle en tant que fixateur pour substances odoriférantes et huiles de parfum.

13. Utilisation de nonanoate de cétyle, de nonanoate de stéaryle ou de mélanges de nonanoate de cétyle et de nonanoate de stéaryle en tant qu'agent pour
- réduire l'évaporation de substances odoriférantes dans une composition de substances odoriférantes, en particulier immédiatement après l'application;
- la stabilisation dans le temps du profil olfactif d'une composition de substances odoriférantes;
- l'augmentation ou la stabilisation dans le temps de l'impact olfactif d'une composition de substances odoriférantes;
- le prolongement de l'adhésion de substances odoriférantes d'une composition de substances odoriférantes, en particulier de la note de tête et/ou note de base, à la peau et/ou aux cheveux, en particulier à la peau humaine;
et/ou
- pour conférer ou renforcer une sensation agréable sur la peau d'une composition de substances odoriférantes.
